(19) Europäisches Patentamt
European Patent Office
Office européen des brevets

(11) Veröffentlichungsnummer: **0 225 602 B1**

(12) **EUROPÄISCHE PATENTSCHRIFT**

(45) Veröffentlichungstag der Patentschrift: **09.10.91**

(51) Int. Cl.5: **C07C 321/18**, C07C 323/00, A61K 31/19, A61K 31/215, C07K 5/06, A61K 37/02

(21) Anmeldenummer: **86116810.2**

(22) Anmeldetag: **03.12.86**

(54) **Alpha-Hydroxythioether.**

(30) Priorität: **06.12.85 CH 5228/85**

(43) Veröffentlichungstag der Anmeldung:
**16.06.87 Patentblatt 87/25**

(45) Bekanntmachung des Hinweises auf die Patenterteilung:
**09.10.91 Patentblatt 91/41**

(84) Benannte Vertragsstaaten:
**AT BE CH DE ES FR GB GR IT LI LU NL SE**

(56) Entgegenhaltungen:
**EP-A- 0 063 858**
**EP-A- 0 121 350**
**EP-A- 0 123 543**
**EP-A- 0 170 048**

**TETRAHEDRON LETTERS, Band 21, Nr. 33, 1980, Seiten 3143-3146, Pergamon Press Ltd, GB; E.J. COREY et al.: "Total synthesis of slow reacting substances (SRS). "Leukotriene C-2" (11-trans-leukotriene C) (3) and leukotriene D (4)"**

(73) Patentinhaber: **CIBA-GEIGY AG**
**Klybeckstrasse 141**
**CH-4002 Basel(CH)**

(72) Erfinder: **Beck, Andreas, Dr.**
**Reutebachgasse 20A**
**W-7800 Freiburg(DE)**
Erfinder: **Breitenstein, Werner, Dr.**
**St. Galler-Ring 179**
**CH-4054 Basel(CH)**
Erfinder: **von Sprecher, Andreas, Dr.**
**Nelkenweg 5**
**CH-4104 Oberwil(CH)**
Erfinder: **Lang, Robert Werner, Dr.**
**Hagenbachweg 10**
**CH-4133 Pratteln(CH)**
Erfinder: **Oertle, Konrad, Dr.**
**Vogesenstrasse 10**
**CH-4106 Therwil(CH)**

(74) Vertreter: **Zumstein, Fritz sen., Dr. et al**
**Bräuhausstrasse 4**
**W-8000 München 2(DE)**

**EP 0 225 602 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft Verbindungen der Formel

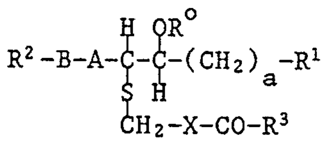

(I)

worin $R^0$ Wasserstoff oder $C_1$-$C_7$-Alkanoyl bedeutet, $R^1$ $C_1$-$C_3$-Alkyl, welches am endständigen C-Atom durch Hydroxy, $C_1$-$C_7$-Alkanoyloxy, Benzoyloxy, Halogen der Atomnummer bis und mit 17 oder Methoxy substituiert sein kann, oder $C_1$-$C_3$-Perfluoralkyl darstellt, $R^2$ einen aliphatischen Rest mit 5 bis 15 C-Atomen bedeutet, $R^3$ Hydroxy, $C_1$-$C_7$-Alkoxy oder eine Gruppe der Formel

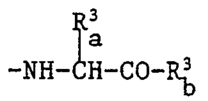

(Ia)

darstellt, in der $R^3_a$ Wasserstoff oder $C_1$-$C_5$-Alkyl und $R^3_b$ Hydroxy, $C_1$-$C_7$-Alkoxy oder Amino bedeutet, A Ethylen bedeutet oder, falls $R^1$ einen halogenierten Rest und/oder B Phenylen darstellt, Vinylen oder eine Einfachbindung darstellt, B Phenylen oder eine Einfachbindung bedeutet, a für eine ganze Zahl von 1 bis 7 steht und X eine Einfachbindung, die Methylengruppe oder eine unsubstituierte oder durch den Acylrest einer gegebenenfalls mono- oder polyhalogenierten $C_1$-$C_5$-Alkansäure oder durch $\gamma$-Glutamyl N-substituierte Aminomethylengruppe bedeutet, mit der Massgabe, dass $R_1$ von Hydroxymethyl verschieden ist, wenn B eine Einfachbindung bedeutet, sowie Salze solcher Verbindungen mit salzbildenden Eigenschaften.

Die Raumdarstellung in der obigen Formel I ist für die bevorzugten Vebindungen, in welchen das O-Atom der Hydroxylgruppe mit dem S-Atom in der relativen trans-Konfiguration sind, so zu verstehen, dass die Symbole der ersten Zeile oberhalb, die der dritten Zeile dann unterhalb der Darstellungsebene liegen (oder umgekehrt), was für die abgebildete Formel der gegenseitigen Konfiguration (RS)-(SR) beider centralen Kohlenstoffatome gemäss der Kahn-Ingold-Prelog-Konvention entspricht.

Gegenstand der Erfindung bilden auch Verfahren zur Herstellung der oben definierten erfindungsgemässen Verbindungen, sowie pharmazeutische Zusammensetzungen, welche diese Verbindungen als Wirkstoff enthalten, und entsprechende Herstellungsverfahren, mit welchen solche Zusammensetzungen auf nicht-chemischem Wege hergestellt werden. - Ein weiterer Gegenstand der Erfindung ist die therapeutische Anwendung der oben definierten Verbindungen und pharmazeutischen Zusammensetzungen, insbesondere bei Linderung und Behebung solcher krankhafter Zustände, bei welchen die ausgeprägte Leukotrien-antagonisierende und/oder Phospholipase-hemmende Wirksamkeit der erfindungsgemässen Verbindungen zur Geltung kommt, wie bei Allergien verschiedener Art, vor allem bei Asthma, bzw. bei Entzündungen, vor allem der Haut und Schleimhäute.

Vor einigen Jahren wurde nachgewiesen, vgl. H.R. Morris et al. Nature 285, 1045-106 (May 1980) und L. Oerning, S. Hammarström und B. Samuelsson: Proc. Natl. Acad. Sci. USA 77 (4), 2014-2017 (1980), dass Leukotriene, insbesondere Leukotrien C und D, mit höchster Wahrscheinlichkeit als primäre Vermittler einer sofort einsetzenden Ueberempfindlichkeits-Reaktion für die Bronchokonstriktion bei Asthma verantwortlich sind.

Das strukturelle Grundgerüst der Leukotriene allgemein wird durch eine mehrfach ungesättigte, lineare Eicosansäure gebildet, die charakteristische Substituenten in 1-, 5- und 6-Stellung trägt, wie es für die erwähnten wichtigsten Vertreter formelmässig abgebildet wird:

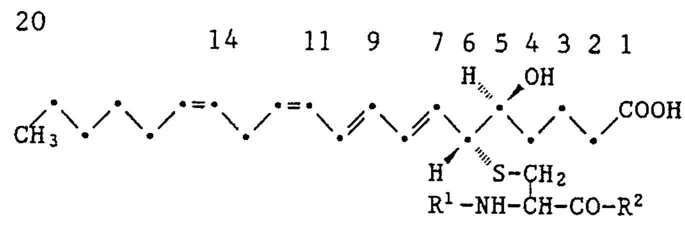

LTC-4: $R^1$ = $HOCOCH(NH_2)CH_2CH_2CO$- ; $R^2$ = $-NHCH_2COOH$
LTD-4: $R^1$ = H- ; $R^2$ = $-NHCH_2COOH$
LTE-4: $R^1$ = H- ; $R^2$ = -OH

[Hier ist die Raumdarstellung so zu verstehen, dass die ganze olefinische Kette in der Darstellungsebene liegt und die mit Pfeil angegebenen Valenzstriche sich oberhalb der Darstellungsebene, die punktierten dagegen unterhalb der Ebene befinden.]

In ihren physiologischen Eigenschaften zeichnen sich Leukotriene im allgemeinen dadurch aus, dass sie eine markante Kontraktion von glatten Muskeln verschiedenster Art verursachen. Vom Standpunkt der Gesundheit ist ein solcher Effekt meistens unerwünscht, und dementsprechend steht die Suche nach geeigneten Leukotrien-Antagonisten im Vordergrund der Forschung auf diesem Gebiet.

In der EP-A-123543 sind Homologe und Derivate LTC-4, LTD-4 und LTE-4 mit Leukotrien-antagonistischer Wirkung beschrieben. Unter den Offenbarungsumfang fallen auch Leukotrienderivate, die anstelle der terminalen Carboxygruppe Hydroxymethyl oder einen von Carboxy verschiedenen sauren Rest aufweisen.

Ueberraschenderweise hat sich nun gezeigt, dass die erfindungsgemässen Verbindungen der Formel I, obwohl sie mehrere Strukturmerkmale mit bekannten Leukotrienen gemeinsam haben, diesen gegenüber eine ausgeprägte antagonistische Wirkung ausüben. So wirken sie in verschiedenen Testanordnungen in vitro deutlich Leukotrien-antagonisierend.

So hemmen sie z.B. im getesteten Konzentrationsbereich von etwa 0,1-25 µMol/l die durch Leukotrien-$D_4$ ($LTD_4$ - siehe oben) induzierte Kontraktion eines glatten Muskels. Dieser sogenannte $LTD_4$-Antagonismuswird experimentell z.B. folgendermassen ermittelt: In Segmenten, die dem Ileum eines 300-400 g schweren Meerschweinchens entnommen wurden und in einem Organbad in Tyrode-Lösung bei 38° C und unter Begasung mit einem Gemisch von 95 % Sauerstoff und 5 % Kohlenstoffdioxid bei einer Belastung von 1 g inkubiert wurden, werden mit synthetischem Leukotrien $D_4$ (als Kaliumsalz) Kontraktionen ausgelöst und isotonisch registriert. Das Ausmass der Hemmung durch die Prüfsubstanz wird nach einer Vorinkubation von 2 Minuten ermittelt und als $IC_{50}$, d.h. die Konzentration, welche die Testkontraktion um 50 % reduziert, ausgewertet. Der $LTD_4$-Antagonismus kann auch in vivo durch Bronchokonstriktions-Standardtest am Meerschweinchen bei Aerosol-Verabreichung nachgewiesen werden. (Die Beschreibung der Testmethode befindet sich im Anhang nach den Beispielen).

Ueberraschenderweise üben Verbindungen der Formel I auch eine ausgeprägte Hemmwirkung auf andere physiologisch wichtige Enzymsysteme aus. So wurde die Hemmung von Phospholipase $A_2$ aus menschlichen Leukocyten im getesteten Konzentrationsbereich von etwa 0,5-50 µMol/l beobachtet. (Die experimentelle Anordnung für diese Bestimmung ist im Anhang nach den Beispielen näher beschrieben.) Ebenfalls wurde die Hemmung von Phospholipase C aus menschlichen Thrombocyten im getesteten Konzentrationsbereich von etwa 1-100 µMol/l beobachtet (für experimentelle Anordnung siehe den Anhang nach den Beispielen).

Die durch diese Methoden in vitro angedeuteten antiallergischen bzw. antiinflammatorischen Eigenschaften werden auch im Tierversuch in vivo bestätigt. So lässt sich die lokale antiinflammatorische Wirksamkeit beispielsweise nach der von G. Tonelli und L. Thibault [Endocrinology 77, 625 (1965)] entwickelten Methode durch Hemmung des mit Crotonöl induzierten Rattenohroedems bei der Normalratte im Dosisbereich von etwa 1 bis etwa 100 mg/ml nachweisen.

Dank diesen wertvollen pharmakologischen Eigenschaften können die erfindungsgemässen Verbindungen der Formel I überall dort therapeutische Anwendung finden, wo die allergogene Wirkung der Leukotriene zu krankhaften Zuständen führt und zu mildern oder zu beheben ist. Demzufolge können sie beispielsweise zur Behandlung allergischer Zustände und Erkrankungen, wie insbesondere Asthma, aber auch Heufieber sowie obstruktiver Lungenkrankheiten einschliesslich zystischer Fibrose, verwendet werden. Ebenfalls sind sie, dank ihrer antiinflammatorischen Wirksamkeit, als entzündungshemmende Mittel, insbesondere als externe (topische) Hautphlogistatika für die Behandlung entzündlicher Dermatosen jeglicher Genese, wie bei leichten Hautirritationen, Kontaktdermatitis, Exanthemen und Verbrennungen, sowie als Schleimhautphlogostatika für die Behandlung von Mukosaentzündungen, z.B. der Augen, Nase, Lippen,

Mund und Genital-, bzw. Analregion, geeignet. Ferner können sie als Sonnenschutzmittel verwendet werden. Die hohe hemmende Wirksamkeit auf verschiedene Blutfaktoren weist zudem auf die Möglichkeit der therapeutischen Anwendung der Verbindungen der Formel I im Indikationsbereich Thrombose und Blutgerinnung hin.

Wie bereits oben erwähnt wurde, ist im Strukturaufbau der erfindungsgemässen Verbindungen der Formel I und Leukotrienen allgemeine Analogie zu finden, insbesondere in der eingangs definierten bevorzugten trans-Konfiguration des vicinalen S- und O-Atoms und der gesamten Struktur des Mercaptoalkansäure-Rests M (insbesondere in seiner typischen Form eines Cystein-Peptids). Von Leukotrienen unterscheiden sie sich dagegen wesentlich dadurch, dass bei ihnen im linearen Rest (L) die charakteristische terminale Carboxylgruppe fehlt und durch verschiedene andere funktionelle Gruppen, wie insbesondere Halogene, gegebenenfalls ersetzt werden kann. Im Unterschied zu Leukotrienen ist bei ihnen auch die Anzahl, Charakter und Raumordnung der Mehrfachbindungen nicht wesentlich, da sie sogar fehlen oder durch Phenylenreste ersetzt werden können. Auch die Gesamtlänge des Rests (L) ist für die Wirksamkeit in breiten Grenzen nebensächlich, und nicht einmal die absolute, und sogar die relative Konfiguration beider oben diskutierten asymmetrischen C-Atome ist für die Wirksamkeit kritisch, wie man es z.B. an wirksamen 5(R),6(S)-Epimeren zeigen kann, welche die gegenüber natürlichen Leukotirenen umgekehrte absolute Konfiguration der Kohlenstoffatome 5 und 6 der Kohlenwasserstoff-Kette (L) hat.

Die in der eingangs definierten Formel I durch Symbol a angegebene Anzahl der Methylengruppen ist vorzugsweise 1 oder 2. Unter bevorzugten Bedeutungen von $R^0$ in Formel I ist in erster Linie Wasserstoff, ferner auch $C_{1-4}$-Alkanoyl, wie Acetyl, zu nennen.

In der oben definierten Formel I steht Symbol $R^1$ vorzugsweise für ein unsubstituiertes oder durch Chlor oder insbesondere Fluor am endständigen C-Atom substituiertes Alkyl, wie Methyl, Propyl und vor allem Ethyl, bzw. Chlormethyl oder Fluormethyl, oder auch für ein entsprechendes $\omega$-Hydroxyalkyl, wie insbesondere $\beta$-Hydroxyethyl, wobei die Hydroxylgruppe nicht nur in freier, sondern auch in veresterter Form vorliegen kann. Die veresterte Hydroxylgruppe ist vorzugsweise durch den Rest einer höchstens 12 C-Atome aufweisenden aliphatischen oder aromatischen Carbonsäure, wie Benzoesäure oder insbesondere einer $C_{1-7}$-Alkansäure, vor allem Essigsäure, verestert. Als ein Perfluoralkyl ist Trifluormethyl bevorzugt.

Der durch Symbol $R^2$ dargestellte aliphatische Rest ist vorzugsweise ein linearer Rest, z.B. ein Alkylrest, bestehend aus 5-15, vorzugsweise 7-12 C-Atomen, wie insbesondere Heptyl, Nonyl, Undecyl und Dodecyl, oder ein entsprechender einfach oder mehrfach ungesättigter Rest, der eine, zwei oder drei mehrfache Bindungen, wie Dreifachbindungen und vor allem Doppelbindungen beliebig in cis- oder trans-Konfiguration, in beliebigen Kombinationen trägt. Diese Mehrfachbindungen befinden sich vorzugsweise möglichst nahe dem Schwefelatom, d.h. in $\alpha,\beta$-Stellung zum schwefeltragenden C-Atom bzw. konjugiert mit dem durch A dargestellten Vinylenrest. Bevorzugte Reste $R^2$ dieser Art sind z.B. 1-Alkenyl-, 1,3-Alkadienyl- und 1,3,6-Alkatrienyl-Reste, wie insbesondere 1-Heptenyl, 1-Octenyl, 1-Nonenyl, 1-Decenyl, 1-Undecenyl und 1-Dodecenyl bzw. 1,3-Octadienyl, 1,3-Decadienyl, 1,3-Dodecadienyl sowie 1,3,6-Dodecatrienyl, in welchen allen die Doppelbindungen je einzeln in cis- oder trans-Konfiguration vorliegen und beliebige Kombinationen bilden können.

Der durch Symbol A in Formel I dargestellte Vinylenrest kann in cis- oder trans-Konfiguration vorliegen.

Symbol B in Formel I kann vorzugsweise für Einfachbindung stehen; falls aber bei dieser Bedeutung von B zugleich im Rest $R^1$ kein Halogenatom vorhanden ist, muss A den Ethylenrest bedeuten. Symbol B kann vorzugsweise auch einen Phenylenrest, wie m- und insbesondere o- und p-Phenylenrest, darstellen, welcher durch einen oder auch mehrere $C_{1-4}$-Alkylreste, vor allem Methylreste, die insgesamt höchstens 6 C-Atome zählen, alkyliert sein kann, vorzugsweise aber unsubstituiert ist. Falls B für Phenylen steht, stellt A vorzugsweise die Einfachbindung dar.

Das in der eingangs angegebenen Formel I definierte Symbol $R^3$ bildet zusammen mit der benachbarten Carbonylgruppe -CO- einen freien oder funktionell abgewandelten Carboxylrest; falls $R^3$ für Hydroxyl steht, bildet es mit der Carbonylgruppe den Carboxylrest einer freien Carbonsäure, falls es ein Alkoxy bedeutet, insbesondere ein solches mit höchstens 7 C-Atomen, vor allem Methoxy, ergänzt es einen Carbonsäureester, und falls es für eine Aminogruppe steht, gehört es zur amidischen Bindung eines Carboxamids oder, falls die Aminogruppe geeignet substituiert ist, eines Peptids. Im letztgenannten Fall ist die substituierte Aminogruppe das Grundelement einer Aminosäure, wie einer $\alpha$-Aminocarbonsäure und insbesondere einer $\alpha$-Amino-$C_{2-7}$-Alkansäure, vorzugsweise einer solchen, die in der Natur vorkommt, wie Leucin, Valin, Alanin (insbesondere in der "natürlichen" L-Form) und vor allem Glycin. Das Carboxyl dieser Aminosäuren kann dann wiederum als freies Carboxyl vorliegen oder in der oben definierten Weise als eine Estergruppe, wie insbesondere ein $C_{1-7}$-Alkoxycarbonyl, oder die Carboxamidogruppe $-CONH_2$ funtkionell abgewandelt sein. Derartige bevorzugte Bedeutungen des Symbols $R^3$ entsprechen dann der Partialformel

$$-NH-\underset{|}{\overset{R_a^3}{C}}H-CO-R_b^3 \qquad (R_o^3)$$

worin $R_a^3$ ein $C_{1-5}$-Alkyl oder vorzugsweise Wasserstoff und $R_b^3$ Hydroxyl, $C_{1-7}$-Alkoxy oder die primäre Aminogruppe $NH_2$ darstellt.

Das eingangs definierte Symbol -X- kann einerseits eine einfache C-C-Bindung darstellen, und somit zusammen mit den benachbarten Gruppen den Rest der Mercaptoessigsäure $-S-CH_2-CO-R^3$ bilden; in diesem Fall ist unter den obgenannten Bedeutungen von $R^3$ Hydroxyl besonders bevorzugt. Andererseits kann -X- eine gegebenenfalls am Stickstoffatom acylierte Aminomethylengruppe darstellen, die dann der Partialformel

$$R^4-NH-\overset{|}{C}H- \qquad (-X_o-)$$

entspricht, worin $R^4$ Wasserstoff oder den Acylrest einer Carbonsäure bedeutet, wie einer aliphatischen oder aromatischen Carbonsäure mit höchstens 12 C-Atomen, insbesondere einer unsubstituierten oder substituierten, vorzugsweise linearen, $C_{1-5}$-Alkansäure. Unter substituierten Alkansäuren dieser Art sind insbesondere zu nennen: einerseits mono-oder vorzugsweise poly-halogenierte, insbesondere chlorierte oder fluorierte, $C_{1-5}$-Alkansäuren, wie in erster Linie die Trifluoressigsäure, andererseits mono- und dibasische Aminosäuren einschliesslich Monoamide der letzteren, insbesondere $\alpha$-Aminosäuren vom Typ derjenigen, die als Bausteine von Peptiden und insbesondere in L-Form in der Natur vorkommen; unter diesen ist beispielsweise die Glutaminsäure hervorzuheben, die vorzugsweise mit ihre: $\gamma$-Carboxyl die Aminogruppe acyliert. Dieser Darstellung nach bedeutet Symbol $R^4$ vorzugsweise Wasserstoff, Trifluoracetyl oder $\gamma$-Glutamyl der Formel $HOCOCH(NH_2)CH_2CH_2CO-$, wobei beim letzteren sein freies Carboxyl in einer Salzform vorliegen kann.

Vorzugsweise bildet die oben charakterisierte Aminomethylengruppe zusammen mit den benachbarten Symbolen einen gegebenenfalls acylierten Cystein-Rest der Partialformel

$$R^4-NH-\underset{}{\overset{-S-CH_2}{\overset{|}{C}}}H-CO-R^3 \qquad \text{bzw. in Kurzform} \quad R^4-\overset{|}{Cys}-R^3 \; ,$$

worin $R^3$ und $R^4$ die obgenannten allgemeinen und bevorzugten Bedeutungen haben, wobei der L-Cysteinyl-Rest mit der in der Natur vorkommenden Konfiguration am asymmetrischen C-Atom bevorzugt ist. In diesem Fall bedeutet $R^3$ vorzugsweise Hydroxyl, $C_{1-4}$-Alkoxy oder einen am Stickstoff gebundenen, gegebenenfalls durch ein $C_{1-4}$-Alkanol veresterten Glycinrest, und $R^4$ insbesondere Wasserstoff, Trifluoracetyl oder $\gamma$-Glutamyl (auch in Salzform).

Die meisten Verbindungen der Formel I können, ihrem individuellen Charakter nach, auch in Form von Salzen vorliegen. Diejenigen davon, welche eine genügende Acidität haben, wie insbesondere die mit freien Carboxylgruppen, können Salze mit Basen, wie insbesondere anorganischen Basen, vorzugsweise physiologisch verträgliche Alkalimetall-Salze, vor allem Natrium- und Kalium-Salze, bilden. Diejenigen der Verbindungen der Formel I, welche eine genügende Basizität haben, wie Ester und Amide von Aminosäuren, können als Säureadditionssalze, insbesondere als physiologisch verträgliche Salze, mit üblichen pharmazeutisch anwendbaren Säuren vorliegen; von anorganischen Säuren sind die Halogenwasserstoffsäuren, wie die Chlorwasserstoffsäure, sowie Schwefelsäure und Phosphor- bzw. Pyrophosphorsäure besonders zu nennen, von den organischen Säuren sind es in erster Linie die Sulfonsäuren, z.B. die aromatischen, wie Benzol- oder p-Toluol-sulfonsäure, Embonsäure und Sulfanilsäure, oder Niederalkansulfonsäuren, wie Methansulfon-, Ethansulfon-, Hydroxyethansulfon- sowie Ethylendisulfonsäure, oder aber auch aliphatische, alicyclische, aromatische oder heterocyclische Carbonsäuren, wie Ameisen-, Essig-, Propion-, Bernstein-, Glykol-, Milch-, Aepfel-, Wein-, Zitronen-, Fumar-, Malein-, Hydroxymalein-, Oxal-, Brenztrauben-, Phenylessig-, Benzoe-, p-Aminobenzoe-, Anthranil-, p-Hydroxybenzoe-, Salicyl- und p-Aminosalicylsäure, sowie Ascorbinsäure. Verbindungen der Formel I, die sowohl basische wie saure funktionelle Gruppen, wie freie Carboxyl- und Aminogruppen, enthalten, können auch als innere Salze vorliegen.

Ganz besonders hervorzuheben sind Verbindungen der Formel I, in welchen der ganze Rest (M) der eingangs erwähnten Mercaptoalkancarbonsäure durch eine der folgenden Formeln dargestellt ist, wobei die Aminosäurereste der "natürlichen" L-Reihe bevorzugt sind:

$$HOCO-CH(NH_2)-CH_2-CH_2-CO-NH-CH(CH_2-S-)-CO-NH-CH_2-COOH \quad (M-1),$$

bzw. in Kurzform

H-Glu-OH ┌ Cys-Gly-OH

$CF_3-CO-NH-CH(CH_2-S-)-CO-NH-CH_2-COOH$ bzw. in Kurzform $CF_3CO-Cys-Gly-OH$ (M-2),

$CF_3-CO-NH-CH(CH_2-S-)-COOH$ bzw. in Kurzform $CF_3CO-Cys-OH$ (M-3),

$NH_2-CH(CH_2-S-)-CO-NH-CH_2-COOH$ bzw. in Kurzform $H-Cys-Gly-OH$ (M-4),

$NH_2-CH(CH_2-S-)-COOH$ bzw. in Kurzform $H-Cys-OH$ (M-5)

sowie $-S-CH_2-COOH$ (M-6).

Mitinbegriffen sind auch entsprechende Verbindungen, worin die Carboxylgruppen in Form eines primären Amids oder $C_{1-4}$-Alkylesters, oder insbesondere eines Salzes, vorzugsweise eines Alkalimetallsalzes, vorliegen.

Besonders bevorzugt sind Verbindungen der Formel I, worin die allgemeinen Symbole die folgenden hervorgehobenen Bedeutungen haben:

a ist eine ganze Zahl von 2 bis 5, $R^0$ ist Wasserstoff, $R^1$ ist Chlormethyl, Fluormethyl oder insbesondere Methyl, $R^2$ ist ein Alkyl mit 5 bis 15, vorzugsweise 8 bis 12, C-Atomen, A ist eine Einfachbindung, B ist ein Phenylen, wie insbesondere das o- oder p-Phenylen, $R^3$ ist Hydroxyl oder -Gly-OH (d.h ein Rest der Formel $-NH-CH_2-COOH$) und -X- ist Methylen oder der oben definierte Rest $-X_o-$, in welchem $R^4$ Trifluoractyl bedeutet und die Trifluoracetylaminogruppe dieselbe Konfiguration wie im natürlichen L-Cystein hat; ganz besonders bevorzugt unter diesen Verbindungen, worin der oben definierte, als M bezeichnete Rest der Mercaptoalkansäure der Formel $-S-CH_2-CH_2-COOH$ oder der oben angegebenen Formel M-2 entspricht. Alle diese bevorzugten Verbindungen können in Form einer freien Säure, oder insbesondere in der Salzform, wie in Form eines physiologisch verträglichen Salzes, z.B. Natrium- oder Kaliumsalzes, vorliegen.

In erster Linie sind die in den Beispielen beschriebenen Verbindungen der Formel I hervorzuheben.

Die erfindungsgemässen Thioether können in an sich bekannter Weise hergestellt werden, beispielsweise dadurch, dass man ein dem eingangs definierten Rest (L) entsprechendes aliphatisches cis-oder vorzugsweise trans-Epoxid von mindestens 11 C-Atomen, insbesondere ein solches der Formel

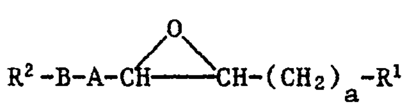

(II)

worin a, A, B, $R^1$ und $R^2$ die oben genannten Bedeutungen haben und worin vorugsweise beide Wasserstoffe am Oxiran-Ring gegeneinander trans orientiert sind, und in welchem eine gegebenenfalls vorhandene Hydroxylgruppe in einer geschützten Form vorliegen kann, mit einer dem oben definierten Rest (M) entsprechenden Mercaptoalkancarbonsäure, insbesondere einer der Formel

$HS-CH_2-X-CO-R^3$ (III)

worin $R^3$ und -X- die obengenannten Bedeutungen haben, in welcher Säure eine gegebenenfalls vorhandene Aminogruppe in einer geschützten Form vorliegen kann, oder mit ihrem Salz oder ihrem Derivat mit umgewandelter Carboxylgruppe umsetzt und wenn notwendig oder erwünscht, eine erhaltene Verbindung der Formel I, worin $R^0$ Wasserstoff bedeutet, zu einer entsprechenden Verbindung, worin $R^0$ ein $C_{1-7}$-Alkanoyl ist, acyliert, und/oder die Schutzgruppen der Hydroxyl- und/oder Aminogruppe abspaltet, und/oder eine als Ester vorliegende Verbindung zur freien Säure oder einem Salz davon verseift und, wenn erwünscht, eine erhaltene freie Verbindung mit salzbildenden Eigenschaften in ein Salz davon oder ein erhaltenes Salz in eine freie Verbindung überführt.

Die Umwandlung erfolgt unter an sich bekannten Bedingungen bei Temperaturen von etwa -20 °C bis etwa +50 °C, vorzugsweise bei Raumtemperatur, und vornehmlich in einem basischen Milieu, beispielsweise in Gegenwart eines Amins, insbesondere eines tertiären aliphatischen, arylaliphatischen oder gesättigten heterocyclischen Amins, wie Trialkylamin (z.B. Triethylamin, oder Ethyl-diisopropylamin), Dialkyl-benzylamin (z.B. N,N-Dimethylbenzylamin), N,N-Dialkylanilin (z.B. N,N-Dimethylanilin) bzw. N-Methyl- oder N-Ethylpiperidin oder N,N'-Dimethylpiperazin. Ueblicherwiese wird die Umsetzung in einem indifferenten organischen Lösungsmittel, wie einem Niederalkanol, z.B. Methanol oder Ethanol, durchgeführt.

Die gegebenenfalls durchzuführende nachträgliche Acylierung der im Hauptverfahren gebildeten Hydroxylgruppe, welche zu Verbindungen der Formel I, worin $R^0$ für $C_{1-7}$-Alkanoyl steht, führt, kann in an sich bekannter Weise durchgeführt werden, z.B. durch Behandeln des primären Produkts, worin $R^0$ für Wasserstoff steht, mit der gewünschten Säure, wie beispielsweise der Ameisensäure, oder mit einem geeigneten reaktionsfähigen Säurederivat, insbesondere einem Halogenid (vorzugsweise Chlorid), symmetrischem Anhydrid, gemischtem Anhydrid (insbesondere einem solchen mit Trifluoressigsäure) oder Keten. Als Reaktionsmedium kann man z.B. überschüssiges Acylierungsmittel verwenden, sowie auch neutrale, nicht acylierbare organische Lösungsmittel, wie Kohlenwasserstoffe (z.B. Pentan, Hexan, Cyclohexan), halogenierte Kohlenwasserstoffe (z.B. Methylenchlorid, Chloroform), Ether (z.B. Diethylether, Ethylenglykoldimethylether, Tetrahydrofuran, Dioxan), Säureester (z.B. Ethylacetat) und Säureamide (z.B. Acetamid, Dimethylformamid); und gegebenenfalls auch nicht acylierbare organische Basen verschiedener Basizität, wie heteroaromatische Basen (z.B. Pyridin, Collidin, Chinolin), tertiäre Amine (z.B. Triethylamin, N-Ethylpiperidin, N-Methylmorpholin, N,N'-Dimethylpiperazin) oder 1,5-Diazabicyclo[5,4,0]undec-5-en; oder aber man arbeitet mit zweckmässigen Kombinationen aller dieser Lösungsmittel. Die Reaktionstemperatur kann sich im Bereich von etwa -70° bis zur Siedetemperatur des Gemisches, vorzugsweise zwischen etwa -20° bis etwa +30 °C, bewegen.

Die Acylierung erfolgt vorzugsweise anschliessend an die Hauptreaktion, d.h. mit Verfahrensprodukten, in welchen die gegebenenfalls vorhandenen Amino- und/oder Hydroxylgruppen noch in geschützter Form vorliegen. (Die Abspaltung der zum vorübergehenden Schutz verwendeten Gruppen erfolgt dann nach an sich bekannten allgemeinen Methoden.) - Man kann jedoch die Acylierung auch in jedem späteren Stadium durchführen, in einem solchen Fall werden aber auch alle anderen freien Hydroxygruppen und Aminogruppen mit demselben Acyl zugleich substituiert.

Wenn im Ausgangsstoff, insbesondere im Substituenten $R^1$ der Formel II ein freies Hydroxyl vorliegt, so kann es während der Umsetzung in einer geschützten, wie veretherten, Form vorliegen. Bevorzugt sind leicht abspaltbare, insbesondere acidolytisch abspaltbare Hydroxyl-Schutzgruppen, wie sie allgemein, insbesondere von der Peptid- und Steroid-Chemie, wohl bekannt sind; dabei werden Schutzgruppen vom Typ tert-Butyl- und insbesondere Tetrahydropyranyl-ether (THP-Ether) besonders bevorzugt. Diese Schutzgruppen können nach erfolgter Hauptreaktion (d.h. Kondensation des Epoxids mit der Mercaptocarbonsäure) in allgemein bekannter Weise, z.B. durch Behandeln mit einer organischen Säure, wie Ameisensäure, Essigsäure, Oxalsäure oder Trifluoressigsäure, oder einem Gemisch davon, und gegebenenfalls in Gegenwart von Wasser und/oder inerter organischer Lösungsmittel, wie Niederalkanole (z.B. Methanol oder Ethanol) und cyclischer Ether (wie Tetrahydrofuran oder Dioxan) unter Freisetzung des Hydroxyls entfernt werden.

Wenn die als Ausgangsstoff eingesetzten Mercaptocarbonsäuren eine freie Aminogruppe enthalten, so kann diese während der Hauptreaktion vorzugsweise in einer geschützten, wie isnbesondere einer acylierten, Form vorliegen. Vorzugsweise werden leicht, insbesondere acidolytisch, abspaltbare Amino-Schutzgruppen angewendet, wie sie allgemein, vor allem in der Peptid-Chemie, einschliesslich Bedingungen für ihre Entfernung wohl bekannt sind. Unter den Amino-Schutzgruppen ist jedoch die Trifluoracetylgruppe besonders hervorzuheben: diese kann nach erfolgter Hauptreaktion im erfindungsgemässen Endstoff weiter bestehen bleiben oder, wenn erwünscht, nachträglich abgespalten werden. Die Abspaltung der N-Trifluoracetylgruppe erfolgt, wie bekannt, vorzugsweise durch Hydrolyse, insbesondere unter basischen Bedingungen, wie mit Alkalimetallcarbonaten (z.B Natrium- oder Kaliumcarbonat) oder verdünnten Alkalilaugen (z.B.

Natrium- oder Kalium-hydroxid) in Amwesenheit von Wasser in einem mit Wasser mischbaren organischen Lösungsmittel, wie einem Niederalkanol (z.B. Methanol oder Ethanol) oder cyclischen Ether (z.B. Tetrahydrofuran oder Dioxan) bei Temperaturen von etwa 0-80 °C, vorzugsweise bei einer leicht erhöhten Temperatur von etwa 50-60 °C. Wenn Estergruppen im zu hydrolysierenden Produkt vorhanden sind, wie eine acylierte Hydroxylgruppe im Hydroxyalkylrest $R^1$ oder eine veresterte Carboxylgruppe im Mercaptosäure-Rest (M), dann werden sie unter diesen Bedingungen gleichzeitig mitverseift.

In der Hauptreaktion (Kondensation mit Epoxid) wird die Mercaptocarbonsäure vornehmlich in Form ihres Esters, vorzugsweise eines $C_{1-4}$-Alkylesters (wie Methyl- oder Ethyl-esters) eingesetzt; falls der erfindungsgemässe Endstoff als freie Säure oder ihr Salz erwünscht ist, dann muss der erhaltene Ester hydrolysiert werden.

Die Hydrolyse wird unter den üblichen Bedingungen durchgeführt, z.B. solchen, die vorangehend für die basenkatalysierte hydrolytische Abspaltung der N-Trifluoracetylgruppe geschildert wurden. Man kann jedoch unter milderen Bedingungen, wie insbesondere bei niedriger Temperatur (vorzugsweise bei Raumtemperatur), mit einer äquivalenten stöchiometrischen Menge Alkali und durch verkürzte Reaktionszeit, gegebenenfalls unter analytischer Kontrolle, z.B. durch Dünnschichtchromatographie, die Estergruppe selektiv unter Erhaltung der N-Trifluoracetylgruppe abspalten, eine acylierte Hydroxylgruppe wird dabei jedoch meistenfalls gleichzeitig abgespalten.

Ausgangsstoffe für das erfindungsgemässe Kondensationsverfahren sind entweder an sich bekannt oder nach bekannten Analogieverfahren in an sich bekannter Weise erhältlich. - So sind z.B. die wichtigen Mercaptocarbonsäuren der Formel III beschrieben (vgl. z.B. E.J. Corey et al.: Tetrahedron Letters 1980, 3143), andere analoge Säuren sind auf dieselbe Weise ausgehend von entsprechenden bekannten Ausgangsstoffen zugänglich. Zur Herstellung von Cysteinderivaten werden vorteilhaft analoge bekannte Cystin-Verbindungen eingesetzt und der üblichen reduktiven Spaltung der disulfidischen Bindung unterworfen oder aber als Cystein-Derivate mit geeignet, z.B. durch Trityl oder Acetylaminomethyl, geschützter Mercaptogruppe verarbeitet.

Das als Ausgangsstoff verwendete cis- oder vorzugsweise trans-Epoxid, z.B. jenes der oben definierten Formel II, kann vornehmlich mittels denselben Verfahren hergestellt werden, welche auch bei der Synthese von Leukotrienen verwendet werden. Bei einem typischen allgemeinen Syntheseweg wird z.B. von einem gesättigten aliphatischen Aldehyd (Alkanal) der Formel

$$O=CH-(CH_2)_a-R^1 \quad (IV)$$

ausgegangen, worin a und $R^1$ die oben angegebenen Bedeutungen haben, wobei eine gegebenenfalls im Rest $R^1$ vorhandene freie Hydroxylgruppe in einer als Ether geschützten, z.B. einer der oben geschilderten, Form vorliegt. Diese Verbindung wird mit Formylmethylentriphenylphosphoran (oder einem äquivalenten Reagens) kondensiert, womit der entsprechende $\alpha,\beta$-ungesättigte Aldehyd, 2-trans-alkenal, der Formel

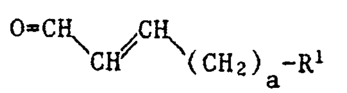

(V)

entsteht, worin a und $R^1$ die oben angegebenen Bedeutungen haben und eine im Rest $R^1$ gegebenenfalls vorhandene freie Hydroxylgruppe als Ether oder Ester geschützt ist. Diese Verbindung wird anschliessend in an sich bekannter Weise, vorzugsweie unter schwach alkalischen Bedingungen (z.B. in Gegenwart von Alkalicarbonaten) mit wässrigem Wasserstoffperoxid epoxidiert, wodurch ein trans-Epoxid, 2-(RS),3(SR)-Epoxy-Alkanal der Formel

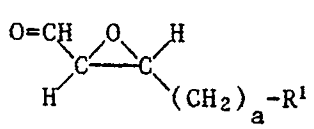

(VI)

resultiert, worin a und $R^1$ die oben angegebenen Bedeutungen haben und eine im Rest $R^1$ gegebenenfalls vorhandene freie Hydroxylgruppe als Ether geschützt ist. Dieser Epoxyaldehyd kann zum gewünschten trans-ungesättigten Epoxid, z.B. dem der oben definierten Formel II, worin eine im Rest $R^1$ gegebenenfalls vorhandene freie Hydroxylgruppe in geschützter, veretherter Form vorliegt und A für den Vinylenrest steht, durch Kondensation mit einem entsprechenden bekannten Benzyliden- bzw. Alkylidentriphenylphosphoran

kondensiert werden. Für mehrfach ungesättigte Epoxide, z.B. diejenigen der Formel II, worin $R^2$ eine oder mehrere Doppelbindungen aufweist, bietet sich eine indirekte Alternative an: anstelle der Wittig-Reaktion mit einem in seiner Kette ungesättigten Yliden-Phoshoran wird der Aldehyd IV zuerst mit $\gamma$-Triphenylphosphoranylidenbutyraldehyd (4-Triphenylphosphoranyliden-butanal) um 4 Kohlenstoffatome verlängert, epoxidiert und erst das erhaltene 6(RS),7(RS)-Epoxy-2-alkenal mit einem einfachen, gesättigten Alkylidentriphenylphosphoran oder einem weniger komplizierten Benzyliden- bzw. Alkenylidentriphenylphosphoran zum gewünschten Epoxid (z.B. einem der Formel II) kondensiert. - Bei Epoxiden der Formel II, worin A eine Einfachbindung und B ein Phenylen ist, wird der Aldehyd IV mit einem entsprechenden Benzylidentriphenylphosporan umgesetzt und anschliessend epoxidiert. In diesem Falle entsteht jedoch meistenfalls ein Gemisch von cis- und trans-Styryl-Derivaten, welches etweder in beide individuelle Isomere aufgetrennt werden muss, oder aber zum Gemisch beider isomerer Epoxide führt, aus welchem dann eventuell im Hauptverfahren 4 Stereoisomere entstehen.

Wenn individuelle Diastereomere erwünscht sind, so kann vorteilhaft an beliebiger Stufe ein individuelles Diastereomeres eines Ausgangsstoffes eingesetzt werden oder aus einem razemischen oder optisch inaktiven Ausgangsstoff durch stereoselektive Reaktionsbedingungen oder optisch aktive Reagentien ein Diastereomeres bevorzugt gebildet werden, oder razemische Diastereomerengemische durch physikalische Trennmethoden, gegebenenfalls unter Anwendung optisch aktiver Hilfsstoffe, in optisch individuelle Diastereomere aufgetrennt werden.

In stereochemischer Hinsicht wird jedoch sowohl die erfindungsgemässe Kondensation der Bildungskomponenten II und III, wie auch die Zubereitung der Ausgangsstoffe vornehmlich so durchgeführt, dass man jeweils stereochemisch einheitliche Ausgangsstoffe einsetzt, die Reaktionen nach Möglichkeit stereoselektiv, z.B. durch Einsatz von optisch aktiven Reagentien und/oder Hilfsstoffen, durchführt und stereochemisch einheitliche Produkte aus Reaktionsgemischen unmittelbar nach der Reaktion isoliert. So werden zB. bei Herstellung der ungesättigten Ausgangsstoffe gegebenenfalls gebildete Isomere mit cis- und trans-Doppelbindungen sofort voneinander getrennt, wozu die üblichen physikalischen Trennungsmethoden, wie insbesondere Chromatographie, geeignet sind. In der Hauptreaktion wird vornehmlich das Epoxid der Formel II als ein individuelles trans-Stereoisomer, jedoch in razemischer Form (wie es normalerweise durch die Epoxydierung eines Olefins gebildet wird) eingesetzt; die Mercaptoalkansäure der Formel III, sofern sie optisch aktiv ist, wird vorzugsweise als ein individuelles optisches Antipode verwendet (was insbesondere bei Cystein und dessen Derivaten der übliche Fall ist) - diese Massnahme ermöglicht, die beiden gebildeten optisch aktiven Diastereomere einfach durch geläufige physikalische Methoden, wie Chromatographie, voneinander zu trennen; falls eine optisch inaktive Mercaptoalkansäure verwendet wird, ist es zur Gewinnung individueller optisch aktiver Produkte unumgänglich, die Methoden der Spaltung in Antipode mittels optisch aktiver Hilfsstoffe, wie z.B. die Bildung von Salzen mit optisch aktiven Basen, anzuwenden. Alle geeigneten Trennungsverfahren sind an sich bekannt und können auch wiederholt oder untereinander zweckmässig kombiniert werden.

Infolge der engen Beziehung zwischen den neuen Verbindungen in freier Form und in Form ihrer Salze sind im voraussgegangenen und nachfolgend unter den freien Verbindungen oder ihren Salzen sinngemäss auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder unter den Reaktionsbedingugnen bildet.

Die bei den erfindungsgemässen Verfahren und ihren Vorstufen auftretenden neuen Ausgangsstoffe und Zwischenprodukte bilden ebenfalls Gegenstand der Erfindung .

Vorzugsweise werden solche Ausgangsstoffe verwendet, und die Reaktionsbedingungen so gewählt, dass man zu den vorstehend als besonders bevorzugt aufgeführten Verbindungen gelangt.

Die vorliegende Erfindung betrifft ebenfalls pharmazeutische Zusammensetzungen und Arzneimittel, welche eine der erfindungsgemässen Verbindungen der Formel I oder ein pharmazeutisch verwendbares Salz davon enthalten. Bei den erfindungsgemässen pharmazeutischen Zusammensetzungen handelt es sich insbesondere um solche, die zur lokalen Verabreichung und vor allem zur Inhalationsverabreichung, z.B. in Form eines Aerosols, mikropulverisierten Pulvers oder einer feinversprühten Lösung, an Säuger, vor allem Menschen, bestimmt sind und den Wirkstoff allein oder zusammen mit einem pharmazeutisch anwendbaren Trägermaterial enthalten.

Pharmazeutische Präparate für topische und lokale Verwendung sind z.B. für die Behandlung der Haut Lotionen und Cremen, die eine flüssige oder semifeste Oel-in-Wasser- oder Wasser-in-Oel-Emulsion enthalten, und Salben (wobei solche vorzugsweise ein Konservierungsmittel enthalten). Für die Behandlung der Augen eignen sich Augentropfen, welche die aktive Verbindung in wässriger oder öliger Lösung

enthalten, und Augensalben, die vorzugsweise in steriler Form hergestellt werden. Für die Behandlung der Nase sind Aerosole und Sprays (ähnlich den weiter unten beschriebenen für die Behandlung der Atemwege), grobe Puder, die durch schnelles Inhalieren durch die Nasenlöcher verabreicht werden, und vor allem Nasentropfen, welche die aktive Verbindung in wässriger oder öliger Lösung enthalten, geeignet; für die lokale Behandlung der Mundhöhle eignen sich auch Lutschbonbons, welche die aktive Verbindung in einer im allgemeinen aus Zucker und Gummiarabikum oder Tragaganth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Als pharmazeutische Zusammensetzungen für die Verabreichung in Form von Aerosolen oder Sprays sind geeignete z.B. Lösungen, Suspensionen oder Emulsionen des erfindungsgemässen Wirkstoffs der Formel I mit einem geeigneten pharmazeutisch annehmbaren Lösungsmittel, wie insbesondere Ethanol und Wasser, oder einem Gemisch solcher Lösungsmittel. Sie können nach Bedarf auch andere pharmazeutische Hilfsstoffe, wie nicht-ionische oder anionische oberflächenaktive Mittel, Emulgatoren und Stabilisatoren, sowie Wirkstoffe anderer Art enthalten und vor allem zweckmässig mit einem Treibgas, wie einem inerten Gas unter erhöhtem Druck oder insbesondere mit einer leicht flüchtigen, vorzugsweise unter normalem atmosphärischem Druck unterhalb der üblichen Raumtemperatur (z.B. zwischen etwa -30 und +10°C) siedenden Flüssigkeit, wie einem mindestens teilweise fluorierten polyhalogenierten Niederalkan, oder einem Gemisch solcher Flüssigkeiten, vermischt ist. Derartige pharmazeutische Zusammensetzungen, welche vorwiegend als Zwischenprodukte oder Vorratsgemische für die Herstellung der entsprechendenden Arzneimittel in fertiger Form verwendet werden, enthalten den Wirkstoff üblicherweise in einer Konzentration von etwa 0,1 bis etwa 10, insbesondere von etwa 0,3 bis etwa 3 Gewichts-%. - Zur Herstellung von Arzneimitteln in fertiger Form wird eine solche pharmazeutische Zusammensetzung in geeignete Behälter, wie Flacons und Druckflaschen, abgefüllt, welche mit einer für solche Zwecke geeigneten Versprühungseinrichtung bzw. Ventil versehen sind. Das Ventil ist vorzugsweise als ein Dosierungsventil konstruiert, welches bei der Betätigung eine vorbestimmte Menge der Flüssigkeit, entsprechend einer vorbestimmten Dosis des Wirkstoffs, freigibt. Bei Herstellung der fertigen Arzneimittelform kann man auch so vorgehen, dass entsprechende Mengen der als Vorratslösung vorliegenden pharmazeutischen Zusammensetzung und des Treibmittels separat in die Behälter abgefüllt werden und erst dort zur Vermischung kommen. Die Dosierung des zu verabreichenden Wirkstoffs der Formel I und die Häufigkeit der Verabreichung hängen ab von der jeweiligen Wirksamkeit bzw. der Länge der Wirkung jeder individueller dieser Verbindungen, von der Stärke der zu behandelnden Krankheit bzw. ihrer Symptome, sowie vom Geschlecht, Alter, Gewicht und individueller Ansprechbarkeit des zu behandelnden Säugers. Im Durchschnitt dürfte die empfohlene tägliche Dosis einer erfindungsgemässen Verbindung der Formel I bei einem 75 kg schweren Säuger (in erster Linie Menschen) im Bereich von etwa 10 bis etwa 500, vorzugsweise zwischen etwa 25 bis etwa 250 mg liegen, wobei die Verabreichung zweckmässig nach Bedarf in mehreren Dosen pro Tag erfolgen kann.

Die Erfindung betrifft auch die Anwendung der erfindungsgemässen Wirkstoffe der Formel I zur Linderung oder Behebung krankhafter Zustände und/oder Symptome des Körpers eines Säugers, insbesondere des Menschen, welche auf die allergogene Einwirkung von Leukotrienen zurückzuführen sind und insbesondere bei Asthma vorkommen. Diese Anwendung bzw. die entsprechende Heilmethode, ist durch die Behandlung des leidenden Körpers bzw. Körperteils mit einer antiallergisch wirksamen Menge einer Verbindung der Formel I allein oder in Form eines Arzneimittels, insbesondere einer zur Inhalation bestimmten pharmazeutischen Zusammensetzung, charakterisiert. Unter der Bezeichnung "eine antiallergisch wirksame Menge" ist eine solche Menge des Wirkstoffes zu verstehen, die zu einer signifikanten Inhibition der durch Leukotriene verursachten Kontraktionen ausreicht.

Die folgenden Beispiele illustrieren näher die vorliegende Erfindung, ohne sie in ihrem Umfang einzuschränken. Alle Temperaturen sind in Celsiusgraden angegeben. Die Aminosäuren als Bildungskomponenten der beschriebenen Verbindungen sind in der "natürlichen" L-Form.

<u>Beispiel 1: 3-[S-4(RS),5(SR)-4-Hydroxy-1,1,1-trifluor-6-cis-eicosen-5-ylthio]-propionsäuremethylester</u>

Zu einer Lösung von 500 mg (1.44 mMol) 4(RS),5(RS)-4,5-Epoxy-1,1,1-trifluor-6-cis-eicosen und 0,62 ml (4,5 mMol) Triethylamin in 8 ml Methanol gibt man die Lösung von 186 mg (1,55 mMol) 3-Mercaptopropionsäuremethylester in 4 ml Methanol. Die Lösung wird 10 Stunden bei Raumtemperatur unter Argon gerührt, das Lösungsmittel im Vakuum abgedampft, und der Rückstand durch Chromatographie an Kieselgel mit Dichlormethan gereinigt. Man erhält die Titelverbindung als hellgelbes Oel.
IR ($CH_2Cl_2$): 2940, 2870, 1745, 1445, 1370, 1300, 1250, 1225, 1150 $cm^{-1}$.

Durch Behandeln derselben Menge des obgenannten Epoxids mit 446 mg (1,55 mMol) N-[N-Trifluoracetylcysteinyl]-glycinmethylester unter analogen Bedingungen wird rohes N-[S-4(RS),5(SR)-4-Hydroxy-1,1,1-trifluor-6-cis-eicosen-5-yl-N-trifluoracetylcysteinyl]-glycinmethylester erhalten, welches durch Chromatographie an Kieselgel und Elution mit einem (19:1)-Gemisch von Chloroform-Methanol gereinigt wird.

IR ($CH_2Cl_2$): 2940, 2870, 1760, 1740, 1700, 1530, 1220, 1180 $cm^{-1}$.

Das als Ausgangsstoff verwendete Epoxid kann folgendermassen hergestellt werden:

a) Die Lösung von 12,6 g (0,1 Mol) 4,4,4-Trifluorbutanal [T. Fuchikami und I. Ojima, J. Am. Chem. Soc. 104, 3527 (1982)] und 30,4 g (0,1 Mol) Formylmethylentriphenylphosphoran [s. Tripett und D.M. Walker, J. Chem. Soc. 1961, 1266)] in 200 ml Dichlormethan wird unter Argon 24 Std. rückfliessend erhitzt. Die rote Lösung wird im Vakuum bei Raumtemperatur vom Lösungsmittel befreit und der Rückstand mit Ether/Hexan (1:1) verrührt. Der feste Anteil wird abfiltriert und dreimal mit Ether/Hexan (1:1) nachgewaschen. Das Filtrat wird im Vakuum eingedampft und der Rückstand mit Dichlormethan an Kieselgel chromatographiert. Das Produkt wird in der zweiten Fraktion ($R_f$ = 0,4) eluiert. Man erhält 6,6,6-Trifluor-2-trans-hexenal als hellgelbes Oel.

IR ($CH_2Cl_2$): 3070, 2960, 2880, 2620, 1700, 1650, 1395, 1250, 1150 $cm^{-1}$.

b) Die Lösung von 5,3 g (0,035 Mol) 6,6,6-Trifluor-2-trans-hexenal in 400 ml Dichlormethan/Methanol (1:1) wird mit 12,7 ml 30-%igem wässrigem Wasserstoffperoxid und mit 425 mg Kaliumcarbonat versetzt und 12 Stunden bei Raumtemperatur gerührt. Man gibt 200 ml Phosphatpuffer (pH = 8) zu und trennt die organische Phase ab. Die wässrige Phase wird noch viermal mit je 50 ml Dichlormethan extrahiert. Die vereinigten organischen Phasen werden über Natriumsulfat getrocknet, über wenig Florisil filtriert und im Vakuum bei Raumtemperatur eingedampft. Man erhält 2(RS),3(SR)-2,3-Epoxy-6,6,6-trifluorhexanal als hellgelbes Oel.

$^1$H-NMR (60 MHz, $CDCl_3$, $\delta$ in ppm): 1,25 (mc, 2H), 1,70-2,60 (m, 2H), 2,90 - 3,50 (m, 2H), 9,05 (d, 1H).

c) Zu einer auf 0 °C gekühlten Lösung von 15,1 g (0,028 Mol) n-Tetradecyltriphenylphosphoniumbromid [E.J. Reist und P.H. Christic, J. Org. Chem. 35, 3521 (1970)] in 100 ml Tetrahydrofuran tropft man 18,8 ml (0,030 Mol) einer 1,6-molaren Lösung von n-Butyllithium in Hexan unter Rühren und unter Argonatmosphäre zu, wobei die Temperatur zwischen 0 und 5 °C gehalten wird. Man bringt die rote Lösung auf Raumtemperatur und rührt noch 30 Minuten. Nach Abkühlen auf -25 °c tropft man die Lösung von 4,6 g (0,027 Mol des gemäss b) erhaltenen 2(RS),3(SR)-2,3-Epoxy-6,6,6-trifluorhexanals in 15 ml Tetrahydrofuran innerhalb 15 Minuten hin. Man lässt die Lösung auf Raumtemperatur erwärmen und rührt noch 2 Stunden. Das Lösungsmittel wird im Vakuum abgedampft und der Rückstand mit Ether versetzt. Man lässt 24 Stunden bei 0 °C stehen, filtriert den ausgefallenen Feststoff ab und dampft das Lösungsmittel im Vakuum ab. Der Rückstand wird mit Dichlormethan/Hexan (1:1, mit 1 % Triethylamin) an Kieselgel chromatographiert. Man erhält das gewünschte 4(RS),5(RS)-4,5-Epoxy-1,1,1-trifluor-6-cis-eicosen als blassgelbes Oel ($R_f$ = 0,45).

IR ($CH_2Cl_2$): 2940, 2870, 1460, 1390, 1300, 1250, 1155 $cm^{-1}$.

Beispiel 2: 3-[S-4(RS),5(SR)-4-Hydroxy-1,1,1-trifluor-6-cis-eicosen-5-ylthio]-propionsäurenatriumsalz

Die Lösung von 580 mg (1,24 mMol) Methylester der Titelverbindung (siehe Beispiel 1) in 40 ml Methanol wird mit 9,5 ml (1,90 mMol) 0,2-N wässriger Natronlauge versetzt und 21 Std. bei Raumtemperatur gerührt. Das Lösungsmittel wird im Vakuum bei 30 °C abgedampft und der Rückstand mit Methanol/Wasser (3:1) an einer Merck Lobar Fertigsäule (Grösse B, LiChroprep RP-8) bei 10 bar chromatographiert. Nach Abdampfen des Laufmittels im Vakuum erhält man die Titelverbindung als farbloses Harz.

$^1$H-NMR (250 MHz, $CD_3OD$, $\delta$ in ppm): 0.9 (t, 3 H), 1,1 - 1,5 (m, 22 H) 1,56 - 1,86 (m, 2H), 2,04-2,5 (m, 4H), 2,4 (t, 2H), 2,7 (mc, 2H), 3,7 (mc, 2H), 5,4 (t, 1H), 5,6 (dt, 1H).

Beispiel 3: 3-[1(RS),2(SR)-2-Hydroxy-1-(2-nonylphenyl)-hexylthio]-propionsäure-methylester

Ein Gemisch von 3,02 g 1(RS),2(RS)-1,2-Epoxy-1-(2-nonylphenyl)-hexan, 3,5 ml 3-Mercaptopropionsäure-methylester, 6 ml Triethylamin und 10 ml Methanol wird während 7 Tage bei Raumtemperatur unter Argon gerührt. Anschliessend wird das Reaktionsgemisch unter vermindertem Druck bei Raumtemperatur eingedampft und der Rückstand durch Chromatographie an Kieselgel mit Hexan und steigendem Anteil an Ether gereinigt. Man erhält 3-[1(RS),2(SR)-2-Hydroxy-1-(2-nonylphenyl)-hexylthio]-propionsäure-methylester als farbloses, zähflüssiges Oel. IR ($CHCl_3$): 3580, 2960, 2930, 2860, 1730, 1440, 1250 $cm^{-1}$.

In analoger Weise, jedoch durch analoges Behandeln des obgenannten Epoxids mit N-(N-Trifluoracetylcysteinyl)-glycinmethylester wird N-[S-1(RS),2(SR)-2-Hydroxy-1-(2-nonylphenyl)-hex-1-yl-N-trifluoracetyl-cysteinyl]-glycinmethylester als zähflüssiges Oel erhalten.

Das als Ausgangsmaterial verwendete 1(RS),2(RS)-1,2-Epoxy-1-(2-nonylphenyl)-hexan kann folgendermassen erhalten werden:

a) 1-(2-Nonylphenyl)-1-hexanol

Ein unter Argonatmosphäre gerührtes Gemisch von 1,1 g Magnesiumspänen, 8 ml Tetrahydrofuran und 3 Tropfen Tetrachlorkohlenstoff wird mit einem Drittel einer Lösung von 11 g 2-Nonylbrombenzol [vgl. EP-OL 0 123 543] in 15 ml Tetrahydrofuran versetzt und während 30 Minuten unter Rückfluss zum Sieden erhitzt. Anschliessend wird die restliche Lösung von 2-Nonylbrombenzol während 35 Minuten zugetropft und das Reaktionsgemisch 2 Stunden am Rückfluss gehalten. Nach Verdünnen mit 15 ml Tetrahydrofuran wird die Suspension auf -10° C abgekühlt und portionenweise zu einer auf -70° C gekühlten Lösung von 4,6 g Hexanal in 12 ml Tetrahydrofuran gegeben. Nach 1-stündigem Rühren bei -70° C wird das Reaktionsgemisch mit 200 ml gesättigter wässriger Ammoniumchloridlösung versetzt, die organische Schicht abgetrennt und die wässrige dreimal mit Ether ausgezogen. Der nach dem Trocknen und Eindampfen der vereinigten Etherextrakte verbliebene Rückstand wird durch Chromatographie an Kieselgel mit Gemischen von Petrolether mit steigendem Anteil an Methylenchlorid gereinigt, womit das gewünschte 1-(2-Nonylphenyl)-1-hexanol als farblose Oel erhalten wird. IR ($CH_2Cl_2$): 3600, 2960, 2925, 2855, 1465 $cm^{-1}$.

b) 1-(2-Nonylphenyl)-1-trans-hexen

Ein Gemisch aus 14,4 g 1-(2-Nonylphenyl)-1-hexanol, 2 g Toluol-4-sulfonsäure-monohydrat und 250 ml Toluol wird während 3 Stunden am Wasserabscheider unter Rückfluss erhitzt. Nach dem Abkühlen wird das Reaktionsgemisch zweimal mit 10%iger (G/V) Natriumbicarbonatlösung und zweimal mit Wasser gewaschen. Die organische Phase wird über Natriumsulfat getrocknet und im Vakuum eingedampft, und der Rückstand durch Chromatographie an Kieselgel und Elution mit Hexan gereinigt. Das gewünschte 1-(2-Nonylphenyl)-trans-hexen wird als blassgelbes Oel erhalten. IR ($CH_2Cl_2$): 2960, 2930, 2855, 1465, 970 $cm^{-1}$.

c) 1(RS),2(RS)-1,2-Epoxy-1-(2-nonylphenyl)-hexan

Eine Lösung von 13,6 g 1-(2-Nonylphenyl)-1-trans-hexen in 350 ml Methylenchlorid wird mit 15,2 g 85%iger 3-Chlorperbenzoesäure versetzt und während 3 Stunden bei Raumtemperatur gerührt. Das Reaktionsgemisch wird mit Methylenchlorid verdünnt und je zweimal mit gesättigter Natriumbicarbonatlösung und Wasser gewaschen. Der nach dem Trocknen und Eindampfen der organischen Phase verbleibende halbfeste Rückstand wird in Hexan aufgeschlämmt und abfiltriert, und das Filtrat unter vermindertem Druck eingedampft. Chromatographische Reinigung des Rohprodukts an Kieselgel mit Hexan/Ether (97:3) ergibt das gewünschte 1(RS),2(RS)-1,2-Epoxy-1-(2-nonylphenyl)-hexan als farbloses Oel. IR ($CH_2Cl_2$): 2960, 2930, 2860, 1470 $cm^{-1}$.

Beispiel 4: 3-[1(RS),2(SR)-2-Hydroxy-1-(2-dodecylphenyl)-pentylthio]-propionsäure-methylester

Ein Gemisch aus 2,5 ml 3-Mercapto-propionsäuremethylester, 4,2 ml Triethylamin, 3,3 g 1(RS),2(RS)-1,2-Epoxy-1-(2-dodecylphenyl)-pentan und 10 ml Methanol wird während 2 Tage unter Argon bei Raumtemperatur gerührt. Das Reaktionsgemisch wird bei 45° C im Vakuum eingedampft und der Rückstand durch Chromatographie an Kieselgel mit Hexan/Ethylacetat (9:1) gereinigt. Man erhält 3-[1(RS),2(SR)-2-Hydroxy-1-(2-dodecylphenyl)-pentylthio]-propionsäure-methylester als farbloses dickflüssiges Oel. IR ($CH_2Cl_2$): 3580, 2930, 2855, 1735, 1465, 1440, 1360 $cm^{-1}$.

Das als Ausgangsmaterial verwendete 1(RS),2(RS)-1,2-Epoxy-1-(2-dodecylphenyl)-pentan erhält man z.B. wie folgt.

a) Cis- und trans-2-(1-Dodecenyl)-brombenzol

Eine auf 5° C gekühlte Suspension von 63,85 g Kalium-tert-butylat in 2,8 l Tetrahydrofuran wird unter Argon innerhalb von 15 Minuten portionenweise mit 291,6 g 2-Brombenzyltriphenyl-phosphoniumbromid [vgl. EP-OL 0 123 543] versetzt, eine Stunde bei 5° C gerührt, mit einer Lösung von 84,8 g Undecanal in 200 ml Tetrahydrofuran tropfenweise behandelt und 24 Stunden bei Raumtemperatur gerührt. Das Reaktionsgemisch wird mit 2 l Ether verdünnt und zweimal mit Wasser gewaschen. Der nach dem Trocknen und Eindampfen der organischen Phase verbleibende halbfeste Rückstand wird in Petrolether aufgeschlämmt, abfiltriert und das Filtrat unter vermindertem Druck eingedampft. Das Rohprodukt wird durch Chromatographie an Kieselgel mit Petrolether gereinigt. Nachfolgende Destillation der reinen Fraktionen im Vakuum ergibt ein Gemisch von cis- und trans-2-(1-Dodecenyl)-brombenzol als farblose Flüssigkeit,

Sdp. 132-135° C/5•$10^{-3}$ mbar. IR ($CH_2Cl_2$): 2925, 2855, 1465, 1020, 970 $cm^{-1}$.

b) 2-Dodecylbrombenzol

Eine Lösung von 108,5 g des gemäss a) erhaltenen Gemisches von cis-und trans-2-(1-Dodecenyl)-brombenzol in 700 ml Ethanol wird mit 1,3 g Platinoxid versetzt und 1 Stunde bei Normaldruck hydriert. Das Reaktionsgemisch wird anschliessend über einen Glasfaserfilter filtriert und im Vakuum eingedampft. Der Rückstand wird in Ether aufgenommen und je zweimal mit gesättigter Natriumbicarbonatlösung und Wasser gewaschen. Der nach dem Trocknen und Eindampfen der organischen Phase erhaltene Rückstand wird im Vakuum destilliert. Man erhält 2-Dodecylbrombenzol als farblose Flüssigkeit, Sdp. 132-135 °C/8•$10^{-3}$ mbar. IR ($CH_2Cl_2$): 2930, 2860, 1470, 1020 $cm^{-1}$.

c) Das letztgenannte Produkt (71,6 g) wird analog Beispiel 3a) zum entsprechenden Grignard-Reagens umgewandelt und mit 25,8 g Valeraldehyd zum 1-(2-Dodecylphenyl)-1-pentanol; IR ($CH_2Cl_2$): 3600, 2930, 2860, 1470, 1040 $cm^{-1}$ ; kondensiert.

d) Die letztgenannte Verbindung wird analog Beispiel 3b) mit 4-Toluolsulfonsäure zum 1-(2-Dodecylphenyl)-1-trans-penten; IR ($CH_2Cl_2$): 2925, 2855, 1465, 970 $cm^{-1}$; dehydratisiert.

e) Ausgehend von 18,5 g 1-(2-Dodecylphenyl)-1-trans-penten und 19,1 g 3-Chlorperbenzoesäure wird analog Beispiel 3c) 1(RS),2(RS)-1,2-Epoxy-1-(2-dodecylphenyl)-pentan erhalten. IR ($CH_2Cl_2$): 2960, 2925, 2850, 1460, 905 $cm^{-1}$.

Beispiel 5: 3-[1(RS),2(SR)-2-Hydroxy-1-(2-nonylphenyl)-hexylthio]-propionsäure-natriumsalz

Ein Gemisch von 3,7 g 3-[1(RS),2(SR)-2-Hydroxy-1-(2-nonylphenyl)-hexylthio]-propionsäure-methylester vom Beispiel 3, 108 ml Tetrahydrofuran und 56,8 ml 0,2N wässriger Natronlauge wird während 16 Stunden bei Raumtemperatur gerührt. Das Reaktionsgemisch wird bei Raumtemperatur unter vermindertem Druck eingedampft, und der Rückstand zwischen Methylenchlorid und 2N Salzsäure verteilt. Die organische Phase wird mit Wasser gewaschen, über Natriumsulfat getrocknet, bei 35 °C im Vakuum eingedampft und der Rückstand durch Chromatographie an Kieselgel mit Methylenchlorid und steigendem Anteil an Aceton gereinigt. Das erhaltene Produkt wird mit 0,1 N wässriger Natronlauge versetzt, bis der pH-Wert der Lösung ca. 7,2 beträgt. Anschliessende Entfernung des Lösungsmittels ergibt die Titelverbindung; IR ($CH_2Cl_2$) 3500, 2960, 2930, 2850, 1590, 1400 $cm^{-1}$.

In analoger Weise, jedoch ausgehend vom N-[S-1(RS),2(SR)-2-Hydroxy-1-(2-nonylphenyl)-hexyl-N-trifluoracetyl-cysteinyl]-glycinmethylester, wird das N-[S-1(RS),2(SR)-2-Hydroxy-1-(2-nonylphenyl)-hexyl-N-trifluoracetyl-cysteinyl]-glycinnatriumsalz erhalten.

Beispiel 6: 3-[1(RS),2(SR)-2-Hydroxy-1-(2-dodecylphenyl)-pentylthio]-propionsäure

Ein Gemisch von 3,76 g 3-[1(RS),2(SR)-2-Hydroxy-1-(2-dodecylphenyl)-pentylthio]-propionsäure-methylester aus Beispiel 4, 50 ml Methanol und 4,25 ml 2N wässriger Natronlauge wird während 20 Stunden unter Argon bei Raumtemperatur gerührt. Anschliessend wird das Reaktionsgemisch unter vermindertem Druck bei 45 °C eingedampft und der Rückstand zwischen Methylenchlorid und 1N Salzsäure verteilt. Die organische Phase wird über Natriumsulfat getrocknet und im Vakuum eingedampft. Der Rückstand, durch Chromatographie an Kieselgel mit Methylenchlorid/Methanol (9:1) gereinigt, ergibt die Titelverbindung in amorpher Form. IR ($CH_2Cl_2$): 3500, 3000 (breit), 2960, 2930, 2860, 1750, 1710, 1470, 1125 $cm^{-1}$.

Beispiel 7: N-[S-1(RS),2(SR)-2-Hydroxy-1-(2-dodecylphenyl)-pentyl-N-trifluoracetyl-cysteinyl]-glycin-methylester

Ein Gemisch aus 3,3 g 1(RS),2(RS)-1,2-Epoxy-1-(2-dodecylphenyl)-pentan, 3,0 g H-(H-Trifluoracetylcysteinyl)-glycinmethylester (E.J. Corey et al., Tetrahedron Lett. 1980, 3193), 4,2 ml Triethylamin und 30 ml absolutem Methanol wird während 20 Stunden unter Argon bei Raumtemperatur gerührt. Nach Filtration wird das Reaktionsgemisch bei Raumtemperatur im Vakuum eingedampft. Chromatographie des Rückstands an Kieselgel mit Methylenchlorid/Aceton (98:2) ergibt die Titelverbindung (Gemisch von Diastereomeren).

IR ($CH_2Cl_2$): 3580, 3390, 2960, 2925, 2845, 1745, 1725, 1685, 1525, 1210, 1170 $cm^{-1}$.

Beispiel 8: N-[S-1(RS),2(SR)-2-Hydroxy-1-(2-dodecylphenyl)-pentyl-N-trifluoracetyl-cysteinyl]-glycin

Ein Gemisch von 4,32 g N-[S-1(RS),2(SR)-2-Hydroxy-1-(2-dodecylphenyl)-pentyl-N-trifluoracetyl-cysteinyl]-glycin-methylester und 50 ml Methanol wird unter Argon mit 3,5 ml 2N wässriger Natronlauge versetzt und 26 Stunden bei Raumtemperatur gerührt. Anschliessend wird das Reaktionsgemisch im Vakuum eingedampft und der Rückstand mit Methylenchlorid/Methanol (9:1) an Kieselgel chromatographiert. Die reinen Fraktionen werden nach dem Eindampfen in Ether aufgenommen und filtriert. Das nach

dem Entfernen des Lösungsmittels verbleibende Gemisch aus der Titelverbindung und dem entsprechenden Natriumsalz wird zwischen Methylenchlorid und 0,2N Salzsäure verteilt. Nach Trocknen und Eindampfen der organischen Phase erhält man N-[S-1(RS),2(SR)-2-Hydroxy-1-(2-dodecylphenyl)-pentyl-N-trifluoracetyl-cysteinyl]-glycin (Gemisch von Diastereomeren).
IR ($CH_2Cl_2$): 3340, 2930, 2855, 1730, 1680, 1530, 1220, 1175 $cm^{-1}$.

Beispiel 9: 3-[1(RS),2(RS)-2-Hydroxy-1-(4-nonylphenyl)-hexylthio]-propionsäuremethylester

Unter Argon werden 0,85 g 1(RS),2(SR)-1,2-Epoxy-1-(4-nonylphenyl)-hexan in 20 ml Methanol gelöst, mit 0,86 g Triethylamin und dann mit 0,44 g 3-Mercaptopropionsäuremethylester versetzt, 6 Tage bei Raumtemperatur gerührt und eingedampft. Chromatographie des Rückstandes an Kieselgel mit Hexan/Ethylacetat (4:1) ergibt die Titelverbindung als farbloses Oel ($R_f$ = 0,5).

Das als Ausgangsmaterial verwendete 1(RS),2(SR)-1,2-Epoxy-1-(4-nonylphenyl)-hexan kann z.B. wie folgt hergestellt werden:

a) 1-(4-Nonylphenyl)-hex-1-en (Gemisch vom cis- und trans-Isomeren). Eine Suspension von 13,9 g Pentyltriphenylphosphoniumbromid in 150 ml Tetrahydrofuran wird unter Argon auf -20° gekühlt, innert 5 Minuten mit 21,2 ml 1,6-M Butyllithiumlösung in Hexan versetzt und weitere 30 Minuten bei 0-10° gerührt. In das auf -60° bis -70° abgekühlte Gemisch wird 6 g 4-Nonylbenzaldehyd in 40 ml Tetrahydrofuran während 30 Minuten zugetropft. Das Reaktionsgemisch wird auf 0-10° spontan erwärmen gelassen, bei dieser Temperatur noch 45 Minuten gerührt und eingedampft. Der Rückstand wird in Hexan/Ethylacetat (1:1) aufgenommen und über Kieselgel filtriert. Das Filtrat wird eingedampft und an Kieselgel mit Hexan chromatographiert. Die Titelverbindung (Gemisch vom cis- und trans-Isomeren) resultiert als farbloses Oel, welches in nächster Stufe direkt eingesetzt wird.

b) 1,2-Epoxy-1-(4-nonylphenyl)-hexan und Trennung in das individuelle cis- [1(RS),2(SR)-] und trans- [1-(RS),2(RS)-] -Isomere. Eine Lösung von 6,32 g 1-(4-Nonylphenyl)-hex-1-en (Gemisch vom cis-und trans-Isomeren) aus vorangehender Stufe in 150 ml Dichlormethan wird unter Kühlung auf 0-5° mit 6,76 g m-Chlorperbenzoesäure (90 % Gehalt) in 100 ml Dichlormethan versetzt und 20 Stunden bei 20° gerührt. Das Reaktionsgemisch wird nacheinander mit 10%iger (G/V) Natriumsulfit-Lösung, 5%iger (G/V) Natriumcarbonat-Lösung und 3 Portionen Wasser gewaschen, über Natriumsulfat getrocknet und eingedampft. Chromatographie des Rückstandes an Kieselgel mit Hexan/Ethylacetat (19:1) ergibt nacheinander das trans-[1(RS),2(RS)-] und das cis- [1(RS),2(SR)-] -Isomere als farblose Oele.

Beispiel 10: 3-[1(RS),2(SR)-2-Hydroxy-1-(2-pentadecylphenyl)-pentylthio]-propionsäure-methylester

Ein Gemisch von 3,0 g 1(RS),2(RS)-1,2-Epoxy-1-(2-pentadecylphenyl)-pentan, 3,6 ml Triethylamin, 1,6 ml 3-Mercapto-propionsäuremethylester, 30 ml Methanol und 2 ml Tetrahydrofuran wird während 12 Stunden untger Argon bei Raumtemperatur gerührt. Anschliessend wird das Reaktionsgemisch während 4 Stunden bei 45°C gerührt. Durch weiteres Vorgehen wie in Beispiel 4 beschrieben erhält man 3-[1(RS),2-(SR)-2-Hydroxy-1-(2-pentadecylphenyl)-pentylthio]-propionsäure-methylester als gelbliches, dickflüssiges Oel. IR ($CH_2Cl_2$): 3580, 2960, 2920, 2850, 1735, 1465, 1435, 1360 $cm^{-1}$.

Das als Ausgangsmaterial verwendete 1(RS),2(RS)-1,2-Epoxy-1-(2-pentadecylphenyl)-pentan erhält man z.B. wie folgt:

a) Cis- und trans-2-(1-Pentadecenyl)-brombenzol

In analoger Weise wie im Beispiel 4a erhält man durch Verwendung von Tetradecanal anstelle von Undecanal die Titelverbindung als farblose Flüssigkeit.
IR ($CH_2Cl_2$): 2930, 2855, 1465, 1020, 970 $cm^{-1}$.

b) 2-Pentadecylbrombenzol

In analoger Weise wie im Beispiel 4b erhält man durch Verwendung von cis- und trans-2-(1-Pentadecenyl)-brombenzol anstelle von cis- und trans-2-(1-Dodecenyl)-brombenzol die Titelverbindung als farbloses Oel.
IR ($CH_2Cl_2$): 2920, 2850, 1470, 1020 $cm^{-1}$.

c) 1-(2-Pentadecylphenyl)-1-pentanol

In analoger Weise wie im Beispiel 3a erhält man ausgehend von 2-Pentadecylbrombenzol und Pentanal die Titelverbindung als blassgelbes Oel.
IR ($CH_2Cl_2$): 3600, 2930, 2860, 1465 $cm^{-1}$.

d) 1-(2-Pentadecylphenyl)-1-trans-penten

In analoger Weise wie im Beispiel 3b erhält man durch Verwendung von 1-(2-Pentadecylphenyl)-1-pentanol anstelle von 1-(2-Nonylphenyl)-1-hexanol die Titelverbindung als farbloses Oel.

IR ($CH_2Cl_2$): 2920, 2855, 1465, 965 $cm^{-1}$.
e) 1(RS),2(RS)-1,2-Epoxy-1-(2-pentadecylphenyl)-pentan
In analoger Weise wie im Beispiel 3c erhält man durch Verwendung von 1-(2-Pentadecylphenyl)-1-trans-penten anstelle von 1-(2-Nonylphenyl)-1-trans-Hexen die Titelverbindung als farbloses Oel.
IR ($CH_2Cl_2$): 2960, 2930, 2860, 1465 $cm^{-1}$.

Beispiel 11: 3-[1(RS),2(SR)-2-Hydroxy-1-(2-pentadecylphenyl)pentylthio]-propionsäure

Ein Gemisch von 1,35 g 3-[1(RS),2(SR)-2-Hydroxy-1-(2-pentadecylphenyl)-pentylthio)-propionsäure-methylester, 10 ml Methanol, 2 ml Tetrahydrofuran und 1,37 ml 2N wässrige Natronlauge wird während 72 Stunden bei Raumtemperatur unter Argon gerührt. Aufarbeitung wie im Beispiel 6 beschrieben ergibt die Titelverbindung als blassgelben Festkörper vom Smp. 28-30°C.
IR ($CH_2Cl_2$): 3580, 3500, 3000 (breit), 2960, 2930, 2860, 1750, 1715, 1465 $cm^{-1}$.

Beispiel 12: N-[S-1(RS),2(SR)-2-Hydroxy-1-(3-nonylphenyl)-hexyl-N-trifluoracetyl-cysteinyl]-glycin-methylester; individuelle Diastereomere 1(R),2(S) und 1(S),2(R)

In analoger Weise wie in Beispiel 3, jedoch ausgehend von 1(RS),2(RS)-1,2-Epoxy-1-(3-nonylphenyl)-hexan und N-(N-Trifluoracetylcysteinyl)-glycin-methylester erhält man die Titelverbindung, welche durch Chromatographie an Kieselgel mit Hexan-Ethylacetat (3:2) in die Diastereomeren aufgetrennt wird. Das 1(R)-,2(S)-Diastereomere, $[\alpha]_D^{20}$ = -78,8±3,1° (0,320 G/V-% in Chloroform) wird vor dem 1(S),2(R)-Diastereomeren, $[\alpha]_D^{20}$ = +97,3±5,4° (0,185 G/V-% in Chloroform) eluiert.

Das als Ausgangsmaterial verwendete 1(RS),2(RS)-1,2-Epoxy-1-(3-nonylphenyl)-hexan kann folgendermassen erhalten werden:
a) 1-(3-Nonylphenyl)-1-hexanol
In analoger Weise wie in Beispiel 3a, jedoch ausgehend von 3-Nonylbrombenzol (vgl. EP-OL 0 123 543) und Hexanol erhält man die Titelverbindung als farbloses Oel.
IR ($CH_2Cl_2$): 3590, 2920, 2930, 1465 $cm^{-1}$.
b) 1-(3-Nonylphenyl)-1-trans-hexen
1-(3-Nonylphenyl)-1-hexanol wird analog Beispiel 3b zur Titelverbindung dehydratisiert.
IR ($CH_2Cl_2$): 2960, 2920, 2850, 1470, 970 $cm^{-1}$.
c) 1(RS),2(RS)-1,2-Epoxy-1-(3-nonylphenyl)-hexan
Umsetzung von 1-(3-Nonylphenyl)-1-trans-hexen analog Beispiel 3c ergibt die Titelverbindung als hellgelbes Oel.

Beispiel 13: N-[S-1(S),2(R)-2-Hydroxy-1-(3-nonylphenyl)-hexyl-N-trifluoracetyl-cysteinyl]-glycin-natriumsalz und dessen 1(R),2(S)-Stereoisomeres

Der entsprechende Ester wird analog Beispiel 2 umgesetzt, und man erhält die Titelverbindung, Smp. 145-146°C.

Auf analoge Weise erhält man aus dem 1(R),2(S)-Ester das N-[S-1(R),2(S)-2-Hydroxy-1-(3-nonylphenyl)-hexyl-N-trifluoracetyl-cysteinyl]-glycin-natriumsalz, Smp. 129-130°C.

Beispiel 14: 3-[3(RS),4(SR)-4-Hydroxy-1-(2-nonylphenyl)-1-trans-octen-3-ylthio]-propionsäuremethylester

Ein Gemisch aus 822 mg 3(RS),4(RS)-3,4-Epoxy-1-(2-nonylphenyl)-1-trans-octen, 0,5 ml 3-Mercaptopropionsäuremethylester, 10 ml Methanol und 1 ml Triethylamin wird während 18 Stunden bei Raumtemperatur unter Argon gerührt. Analoge Aufarbeitung wie im Beispiel 4 beschrieben ergibt die Titelverbindung als farbloses Oel.
IR ($CH_2Cl_2$): 3580, 2960, 2930, 2860, 1735, 1465, 1435, 1360 $cm^{-1}$.

Das als Ausgangsmaterial verwendete 3(RS),4(RS)-3,4-Epoxy-1-(2-nonylphenyl)-1-trans-octen kann beispielsweise nach folgender Vorschrift hergestellt werden:
a) 2-Nonyl-benzaldehyd
Ein unter Argonatmosphäre gerührtes Gemisch von 3,4 g Magnesiumspänen, 25 ml Tetrahydrofuran und 3 Tropfen Tetrachlorkohlenstoff wird mit einem Drittel einer Lösung von 22 g 2-Nonylbrombenzol in 35 ml Tetrahydrofuran versetzt und während 30 Minuten unter Rückfluss zum Sieden erhitzt. Anschliessend wird die restliche Lösung von 2-Nonylbrombenzol während einer Stunde zugetropft und das Reaktionsgemisch 2 Stunden am Rückfluss gehalten. Nach Verdünnen mit 40 ml Tetrahydrofuran wird im Eisbad

auf ca. 5° abgekühlt und während 15 Minuten eine Lösung von 11,6 ml Dimethylformamid in 20 ml Tetrahydrofuran eingetropft. Nach 1-stündigem Rühren bei Raumtemperatur wird das Reaktionsgemisch mit 250 ml gesättigter Ammoniumchloridlösung versetzt, die organische Schicht abgetrennt und die wässrige dreimal mit Ether ausgezogen. Der nach dem Trocknen und Eindampfen der vereinigten Etherextrakte verbliebene Rückstand wird durch Chromatographie an Kieselgel mit Gemischen von Petrolether mit steigendem Anteil an Methylenchlorid gereinigt, womit der gewünschte 2-Nonylbenzaldehyd als blassgelbe Flüssigkeit erhalten wird.

IR ($CH_2Cl_2$): 2920, 2850, 1695, 1600 $cm^{-1}$.

b) Nonyl-benzylalkohol

Eine gerührte Lösung von 9,3 g 2-Nonyl-benzaldehyd in 150 ml Methanol wird während 15 Minuten portionenweise mit 0,57 g Natriumborhydrid versetzt. Nach weiterem Rühren während 30 Minuten wird das Reaktionsgemisch unter vermindertem Druck eingedampft und der Rückstand in Ether aufgenommen. Die organische Phase wird mit eisgekühlter 0,2N-Salzsäure und mit Wasser gewaschen, über Natriumsulfat getrocknet und im Vakuum eingedampft. Chromatographische Reinigung des Rückstands an Kieselgel mit Gemischen von Petrolether mit steigendem Anteil von Ether liefert 2-Nonyl-benzylalkohol als blassgelbes Oel.

IR ($CH_2Cl_2$): 3600, 2925, 2855, 1000 $cm^{-1}$.

c) 2-Nonylbenzylbromid

Zu einem gerührten Gemisch von 6,6 g 2-Nonyl-benzylalkohol und 50 ml Benzol wird während 15 Minuten eine Lösung von 10 g Phosphortribromid in 50 ml Benzol getropft. Das Reaktionsgemisch wird 30 Minuten unter Rückfluss erhitzt und nach dem Abkühlen mit Eiswasser und Ether versetzt. Die organische Phase wird abgetrennt, mit Wasser gewaschen, über Natriumsulfat getrocknet und im Vakuum eingedampft. Chromatographische Reinigung des Rückstands an Kieselgel mit Petrolether liefert 2-Nonylbenzylbromid als farbloses Oel.

IR ($CH_2Cl_2$): 2920, 2850, 1470, 1210 $cm^{-1}$.

d) 2-Nonylbenzyl-triphenylphosphoniumbromid

Ein Gemisch von 7,2 g 2-Nonylbenzylbromid, 5,77 g Triphenylphosphin und 60 ml Toluol wird während 4 Stunden unter Rückfluss erhitzt, abgekühlt und mit 80 ml Ether verdünnt. Der ausgeschiedene 2-Nonylbenzyl-triphenylphosphoniumbromid wird durch Filtration abgetrennt, mit Ether gewaschen und im Vakuum getrocknet; Smp. 174-176°.

e) 3(RS),4(RS)-3,4-Epoxy-1-(2-nonylphenyl)-1-trans-octen

Zu einem unter Argonatmosphäre gerührten und auf 5° gekühlten Gemisch von 5,6 g 2-Nonylbenzyl-triphenylphosphoniumbromid und 50 ml absolutem Tetrahydrofuran werden 6,4 ml einer 1,6M-Lösung von Butyllithium in Hexan gegeben. Nach weiteren 10 Minuten wird innerhalb von 3 Minuten eine Lösung von 2(RS),3(RS)-2,3-Epoxyheptanal in 15 ml Tetrahydrofuran eingetropft. Das Gemisch wird noch eine Stunde bei 5° und 15 Minuten bei Raumtemperatur gerührt, mit Wasser versetzt und dreimal mit Ether extrahiert. Die organische Phase wird über Natriumsulfat getrocknet und eingeengt. Der verbleibende Rückstand wird in Hexan aufgeschlämmt, abfiltriert und das Filtrat unter vermindertem Druck eingedampft. Chromatographische Reinigung des Rückstands an Kieselgel und Eluieren mit einem 97:3-Gemisch (V/V) von Petrolether-Ether ergibt 3(RS),4(RS)-3,4-Epoxy-1-(2-nonylphenyl)-1-trans-octen vom

IR ($CH_2Cl_2$): 2960, 2930, 2860, 1470, 870 $cm^{-1}$.

Beispiel 15: 3-[3(RS),4(SR)-4-Hydroxy-1-(2-nonylphenyl)-1-trans-octen-3-ylthio]-propionsäure

Der Methylester der Titelverbindung wird analog Beispiel 6 umgesetzt. Man erhält die Titelverbindung als Feststoff, Smp. 28-30° C.

IR ($CH_2Cl_2$): 3590, 3000, 2960, 2930, 2860, 1750, 1710, 1470 $cm^{-1}$.

Beispiel 16: N-[S-3(S),4(R)-4-Hydroxy-1-(4-octylphenyl)-octenyl-N-trifluoracetyl-cysteinyl]-glycin-methylester (Gemisch von cis-/trans-Isomeren 60:40)

In analoger Weise wie in Beispiel 3, jedoch ausgehend von 3(R),4(R)-3,4-Epoxy-1-(4-octylphenyl)-1-octen (Gemisch von cis-/trans-Isomeren 60:40) und N-(N-Trifluoracetylcysteinyl)-glycin-methylester erhält man die Titelverbindung (cis-/trans-Isomerengemisch 60:40).

Das als Ausgangsmaterial verwendete 3(R),4(R)-3,4-Epoxy-1-(4-octylphenyl)-1-octen (Gemisch von cis-/trans-Isomeren 60:40) kann folgendermassen erhalten werden:

a) 2-trans-Heptenol

Zu einer Lösung von 10 g Lithiumaluminiumhydrid in 400 ml Ether werden bei 0° C 16,9 g 2-Heptinol in

200 ml Ether innerhalb von 30 Minuten unter Rühren zugetropft, und das entstandene Reaktionsgemisch wird über Nacht unter Rückfluss gekocht. Der Ueberschuss von $LiAlH_4$ wird unter Kühlen in einem Eis-Wasser-Bad durch Zugabe von 40 ml Ethylacetat zerstört und das resultierende Reaktionsgemisch zwischen Ether und kalter 1N Schwefelsäure aufgenommen. Die angesäuerte (pH 2) wässrige Schicht wird noch mit Ether nachextrahiert, die vereinigten organischen Extrakte werden über Magnesiumsulfat getrocknet und im Vakuum eingedampft. Destillation des Rückstandes (18 g) unter vermindertem Druck ergibt 13,2 g 2-trans-Heptenol als farbloses Oel; Sdp. 71-72 °C/13 mbar.

b) 2(R),3(R)-2,3-Epoxyheptanol

Unter wasserfreien Bedingungen wird eine gerührte Lösung von 66,3 ml Tetraisopropylorthotitanat und 38,51 ml D-(-)-Weinsäurediethylester in 1,1 l Methylenchlorid bei -23 °C nacheinander mit 25,7 g 2-trans-Heptenol (siehe oben) und 140 ml einer 3,2M-Lösung von tert-Butylhydroperoxid in Toluol versetzt, 16 Stunden bei -20 °C gehalten und bei -23 °C mit 56 ml 10%iger wässriger L-Weinsäure-Lösung tropfenweise behandelt. Nach weiteren 30 Minuten wird das Gemisch auf +20 °C erwärmen gelassen und noch so lange weiter gerührt, bis sich die organische Schicht klar abtrennen lässt. Diese wird mit 1 l 1%iger wässriger Natriumsulfit-Lösung 1 Stunde gerührt, abgetrennt, mit Wasser gewaschen, über Natriumsulfat getrocknet und im Wasserstrahlvakuum eingeengt. Der Rückstand wird in 1,6 l Diethylether gelöst, auf 0 °C gekühlt, tropfenweise mit 675 ml N-Natronlauge versetzt und 30 Minuten bei 0 °C gerührt. Die abgetrennte organische Phase wird mit gesättigter Natriumchloridlösung gewaschen, getrocknet und eingeengt, womit 2(R),3(R)-2,3-Epoxyheptanol als farblose instabile Flüssigkeit resultiert, welche in der nächsten Stufe sofort verarbeitet wird.

c) 2(S),3(R)-2,3-Epoxyheptanal

Eine Lösung von 13,3 g 2(R),3(R)-2,3-Epoxyheptanol in 100 ml Methylenchlorid wird innert 30 Minuten zu einer gerührten Suspension von 110,1 g Pyridiniumchlorochromat und 41,9 g Natriumacetat in 500 ml Methylenchlorid getropft, wobei die Temperatur durch leichtes Kühlen bei 25 °C gehalten wird. Nach 3 Stunden wird das Reaktionsgemisch mit 500 ml Diethylether verdünnt und über Kieselgel filtriert. Das Filtrat wird mit Phosphatpuffer vom pH 8 gewaschen, über Natriumsulfat getrocknet und eingedampft. Chromatographie des Rückstandes an Kieselgel mit einem Gemisch von Petrolether (Sdp. 30-45 °) und Diethylether (3:2) ergibt 2(S),3(R)-2,3-Epoxyheptanal als farblose Flüssigkeit.

d) 4-Octyl-benzaldehyd

Zu einer auf -25 °C gekühlten Lösung von 45,7 g Octylbenzol in 100 ml Chloroform gibt man unter Argon 68,3 g Titantetrachlorid. Dann tropft man innert 30 Minuten 27,6 g Dichlormethyl-methyl-ether bei -25 °C zu und rührt noch 1,5 Stunden bei dieser Temperatur. Das Reaktionsgemisch wird auf Eiswasser gegossen, die organische Phase abgetrennt und mit Wasser neutral gewaschen. Nach Trocknen über Magnesiumsulfat und Eindampfen im Vakuum wird der Rückstand durch Chromatographie an Kieselgel mit Hexan-Ethylacetat = (19:1) gereinigt. Zunächst wird wenig 2-Octylbenzaldehyd, danach die Titelverbindung eluiert (farbloses Oel).

IR ($CH_2Cl_2$): 2940, 2870, 1700, 1615, 1220, 1175 $cm^{-1}$.

e) 4-Octylbenzylalkohol

Umsetzen von 4-Octylbenzaldehyd analog Beispiel 14b ergibt die Titelverbindung als farblose Flüssigkeit.

IR ($CH_2Cl_2$): 3620, 2950, 2875, 1465, 1010 $cm^{-1}$.

f) 4-Octylbenzylbromid

4-Octylbenzylalkohol wird analog Beispiel 14c umgesetzt. Man erhält die Titelverbindung als farblosen Feststoff.

IR ($CH_2Cl_2$): 2940, 2870, 1520, 1475, 1235, 1210 $cm^{-1}$.

g) 4-Octylbenzyl-triphenylphosphoniumbromid

Umsetzung von 4-Octylbenzylbromid analog Beispiel 14c ergibt die Titelverbindung als Feststoff; Smp. 160-161 °C.

h) 3(R),4(R)-3,4-Epoxy-1-(4-octylphenyl)-1-octen (Gemisch von cis-/trans-Isomeren 60:40)

In analoger Weise wie in Beispiel 9a, jedoch ausgehend von 4-Octylbenzyl-triphenylphosphoniumbromid und 2(S),3(R)-2,3-Epoxyheptanal erhält man die Titelverbindung (cis-/trans-Isomerengemisch 60:40) als hellgelbes Oel.

Beispiel 17: N-[S-3(S),4(R)-4-Hydroxy-1-(4-octylphenyl)-1-octenyl-N-trifluoracetyl-cysteinyl]-glycin-natriumsalz (Gemisch von cis-/trans-Isomeren 60:40)

Das cis-/trans-Isomerengemisch (60:40) der entsprechenden Methylester wird analog Beispiel 2 umgesetzt. Man erhält die Titelverbindung als (cis-/trans Isomerengemisch (60:40); Smp. 120-121 °C.

Beispiel 18: 3-[1(RS),2(SR)-6-Fluor-2-hydroxy-1-(2-nonylphenyl)-hexylthio]-propionsäuremethylester

In analoger Weise wie in Beispiel 4 erhält man durch Verwendung von 1(RS),2(RS)-1,2-Epoxy-6-fluor-1-(2-nonylphenyl)-hexan anstelle von 1(RS),2(RS)-1,2-Epoxy-1-(2-dodecylphenyl)-pentan die Titelverbindung als blassgelbes Oel.
IR ($CH_2Cl_2$): 3580, 2930, 2860, 1735, 1440, 1245 $cm^{-1}$.

Das als Ausgangsmaterial verwendete 1(RS),2(RS)-1,2-Epoxy-6-fluor-1-(2-nonylphenyl)-hexan kann beispielsweise wie folgt hergestellt werden:

a) 1-(2-Nonylphenyl)-1-trans-hexen-6-ol

Eine unter Stickstoffatmosphäre gerührte Lösung von 34,4 g 6-(2-Nonylphenyl)-5-hexensäure-methylester (vgl. EP-OL 0 123 543) in 300 ml absolutem Tetrahydrofuran wird während ca. einer Stunde portionenweise mit 2,8 g Lithiumaluminiumhydrid versetzt. Nach weiteren 10 Minuten werden 30 ml Ethylacetat und anschliessend 30 ml Wasser eingetropft. Das Reaktionsgemisch wird durch Zugabe von 1N Salzsäure angesäuert und mehrmals mit Ethylacetat extrahiert. Durch chromatographische Reinigung des nach dem Trocknen und Eindampfen der vereinigten organischen Phasen verbleibenden Rohprodukts mit Dichlormethan an Kieselgel erhält man die Titelverbindung als gelbliches Oel.
IR ($CH_2Cl_2$): 3620, 2930, 2850, 1470, 970 $cm^{-1}$.

b) 6-Fluor-1-(2-nonylphenyl)-1-trans-hexen

Zu einem unter Argonatmosphäre gerührten und mit einem Eisbad gekühlten Gemisch von 4,68 g Diethylaminoschwefeltrifluorid in 20 ml Dichlormethan wird während 20 Minuten eine Lösung von 8,17 g 1-(2-Nonylphenyl)-1-trans-hexen-6-ol in 20 ml Dichlormethan getropft. Nach weiterem Rühren bei Raumtemperatur während 14 Stunden wird mit Wasser versetzt, die organische Schicht abgetrennt und mit gesättigter Natriumbicarbonatlösung und Wasser gewaschen. Das nach dem Trocknen und Eindampfen der organischen Phase verbleibende Rohprodukt wird durch Flash-Chromatographie an Kieselgel mit Petrolether gereinigt. Man erhält die Titelverbindung als farbloses Oel.
IR ($CH_2Cl_2$): 2930, 2850, 1465, 970 $cm^{-1}$.

c) 1(RS),2(RS)-1,2-Epoxy-6-fluor-1-(2-nonylphenyl)-hexan

In analoger Weise wie in Beispiel 3c erhält man durch Verwendung von 6-Fluor-1-(2-nonylphenyl)-1-trans-hexen anstelle von 1-(2-Nonylphenyl)-1-trans-hexen die Titelverbindung als farbloses Oel.
IR ($CH_2Cl_2$): 2920, 2850, 1455 $cm^{-1}$.

Beispiel 19: 3-[1(RS),2(SR)-6-Fluor-2-hydroxy-1-(2-nonylphenyl)-hexylthio]-propionsäure-natriumsalz

Ein Gemisch von 1,7 g 3-[1(RS),2(SR)-6-Fluor-2-hydroxy-1-(2-nonylphenyl)-hexylthio]-propionsäure-methylester, 5 ml Tetrahydrofuran, 10 ml Methanol und 2,09 ml 2N Natronlauge wird unter Argon 14 Stunden bei Raumtemperatur und 2 Stunden bei 45°C gerührt. Anschliessend wird bei 45°C im Vacuum eingedampft und der Rückstand zwischen Dichlormethan und 1N Salzsäure verteilt. Der nach dem Trocknen und Eindampfen der organischen Phase verbleibende Rückstand wird an Kieselgel mittels Dichlormethan mit steigendem Anteil an Methanol gereinigt. Die erhaltene Säure wird in 10 ml Methanol/Tetrahydrofuran (1:1) aufgenommen, mit einem Aequivalent 1N Natronlauge versetzt und 10 Minuten bei Raumtemperatur gerührt. Der nach dem Entfernen der flüchtigen Anteile verbleibende Rückstand wird zweimal mit Chloroform abgedampft. Man erhält die Titelverbindung als gelblichen amorphen Festkörper.
IR ($CH_2Cl_2$): 2930, 2860, 1600, 1430, 1400 $cm^{-1}$.

Beispiele der pharmazeutischen Zusammensetzungen
und entsprechender fertiger Arzneimittelformen

Unter dem Ausdruck "Wirkstoff" ist nachstehend eine erfindungsgemässe Verbindung der Formel I zu verstehen, insbesondere eine solche, die als Produkt in Beispielen 1-9 beschrieben ist, wie z.B. 3-[S-4(RS)-,5(SR)-4-Hydroxy-1,1,1-trifluor-6-cis-eicosen-5-ylthio]-propionsäure-natriumsalz oder 3-[1(RS),2(SR)-2-Hydroxy-1-(2-nonylphenyl)-hexylthio]-propionsäure-natriumsalz.

Beispiel A: Eine treibmittelhaltige, feststoffaerosolbildende Inhalationssuspension enthaltend 0,1 Gew.-% Wirkstoff

| Zusammensetzung: | Gew.-% |
|---|---|
| Wirkstoff, mikronisiert | 0,1 |
| Sorbitantrioleat | 0,5 |
| Treibmittel A | |
| (Trichlortrifluorethan) | 4,4 |
| Treibmittel B | |
| (Dichlordifluormethan und | 15,0 |
| 1,2-Dichlortetrafluorethan) | 80,0 |

Herstellung: Der Wirkstoff wird unter Feuchtigkeitsausschluss mit Hilfe eines üblichen Homogenisators unter Zusatz des Sorbitantrioleats im Trichlortrifluorethan suspendiert, die Suspension in einen mit Dosierventil versehenen Aerosolbehälter abgefüllt; dieser wird verschlossen und unter Druck mit dem Treibmittel B aufgefüllt.

Beispiel B: Eine zur Inhalation geeignete, etwa 2%ige wässrige Lösung eines Wirkstoffes in Form dessen Natrium- oder Kalium-salzes.

| Zusammensetzung | | |
|---|---|---|
| Wirkstoff (K- oder Na-Salz) | | 2000 mg |
| Ethylendiamintetraessigsäure- | | |
| Dinatriumsalz | | 10 mg |
| Benzalkoniumchlorid | | 10 mg |
| Wasser, frisch destilliert | ad | 100 ml |

Herstellung: Der Wirkstoff wird in etwa 60 ml frisch destilliertem Wasser gelöst und der Stabilisator (Ethylendiamintetraessigsäure-Dinatriumsalz) und das Konservierungsmittel (Benzalkoniumchlorid) hinzugegeben. Nach vollständiger Auflösung aller Komponenten wird die erhaltene Lösung auf 100 ml aufgefüllt, in Druckfläschchen abgefüllt und diese gasdicht verschlossen. Das Treibmittel wird nach Bedarf gasförmig unter Druck oder in flüssiger Form zugegeben.

## ANHANG - PHARMAKOLOGISCHE TESTMETHODEN

### Meerschweinchen-Bronchokonstriktionstest (in vivo, Aerosol):

Man anästhetisiert männliche, 400-700 g schwere Meerschweinchen intraperitoneal mit 1.4 g/kg Urethan und führt eine Polyethylenkanüle in die Vena jugularis ein. Eine zweite Polyethylenkanüle wird in die Trachea eingeführt. Mittels einer in die Speiseröhre eingeführten Kanüle, welche mit einem Statham-Druck-Transduktor verbunden ist, wird der Druck in der Speiseröhre aufgezeichnet. Das Tier wird in eine luftdicht verschliessbare Plexiglaskammer gelegt, die mit einer Fleisch'schen Röhre Nr. 000 und einem Validyne-Transducer MP 45-1 verbunden ist. Mit dieser Anordnung wird der Flow gemessen.

Nach der chirurgischen Präparierung der Versuchstiere wartet man eine gewisse Zeit, damit die pulmonalen Funktionen sich stabilisieren können. Die zu testende Verbindung wird anschliessend gemäss dem nachfolgenden Protokoll verabreicht. Die Versuchstiere werden während einer Minute einer 1-prozentigen Aerosollösung der zu testenden Verbindung (Gew/Vol) oder destilliertem Wasser (zu Kontrollzwecken) ausgesetzt. Für alle Testverbindungen, welche durch Inhalation verabreicht werden, verwendet man ein Monaghan-Ultraschall-Sprühgerät (Modell 670) dessen Partikelgrösse sich zwischen 1 und 8 Mikron bewegt mit einem Hauptanteil von 3 Mikron.

Wässrige Lösungen werden jeweils frisch hergestellt und mit einem On-stream drug vial in die Kammer des Sprühgeräts eingeführt. Der produzierte Sprühnebel wird den Versuchstieren über eine Glaskammer

von 65 ml Inhalt, die mit einer Kanüle mit der Trachea verbunden wird, verabreicht. Nach Ablauf der Behandlungszeit wird mit einem zweiten Monaghan-Ultraschall-Sprühgerät (Modell 670) und über eine gleiche Glaskammer $LTD_4$ (0,3 µg/ml) während 2 Minuten verabreicht.

Es wird die Abnahme der Compliance in der 3. Minute nach $LTD_4$-Applikation abgelesen und zwar wird der Mittelwert von drei Tieren mit dem Mittelwert von drei Kontrolltieren verglichen und die prozentuale Hemmung der Compliance nach folgender Formel errechnet:

$$\% \text{ Hemmung} = 100 - \frac{(100 - \text{Compliance Präparat}) \cdot 100}{(100 - \text{Compliance Kontrolle})}$$

Werden unterschiedliche Wirkstoffkonzentrationen untersucht, so wird die prozentuale Hemmung für jede Konzentration aufgezeichnet, und zwar wird der log Konzentration auf der Abszisse gegen die prozentuale Hemmung auf der Ordinate aufgetragen. Die $IC_{50}$ wird dann durch lineare Regressionsanalyse ermittelt.

In vitro-Test zur Bestimmung der Hemmung der Phospholipase $A_2$ aus menschlichen Leukozyten

Neutrophile polymorphkernige menschliche Leukozyten werden ausgehend von "Buffy coats" durch mehrstufige fraktionierte Sedimentation isoliert und tiefgefroren. Die Phospholipase $A_2$ wird aus der Zellsuspension durch Homogenisieren unter Zusatz von eiskalter 0.36 N $H_2SO_4$ in 2N NaCl extrahiert und der nach Zentrifugation bei 10'000 x g erhaltene Ueberstand gegen Natriumacetatpuffer pH 4.5 dialysiert

Für die Bestimmung der Enzymaktivität wird Enzym (10-30 µg Protein) in 0.1 M Tris/HCl-Puffer pH 7 unter Zusatz von 1 mM $CaCl_2$ und Substrat, bestehend aus biosynthetisch mit $^{14}C$-Oelsäure radioaktiv markiertem Phospholipiden (2 µM) von Escherichia coli bei 37° während 1 Stunde inkubiert. Die Reaktion wird abgestoppt durch Zugabe von Dole-Reagens (Isopropanol/Heptan/1N $H_2SO_4$ 40:10:1, v/v) und die durch Phospholipase $A_2$ selektiv freigesetzte $^{14}C$-Oelsäure extrahiert. Ebenfalls mitextrahiertes Substrat wird durch Filtration des Extraktes durch eine Säule von Kieselgel vollständig entfernt. Die Bestimmung der $^{14}C$-Oelsäure im Eluat erfolgt durch Radiometrie.

Zur Ermittlung einer Hemmwirkung von Prüfsubstanzen auf die Phospholipase $A_2$ werden diese als Lösungen in Wasser, Dimethylsulfoxid (Endkonzentration im Ansatz bis 5 %, v/v) oder Ethanol (Endkonzentration im Ansatz bis 2.5 % v/v) dem Inkubationsumsatz zugesetzt. Die Wirkungsstärke der Prüfsubstanzen wird ausgedrückt durch die $IC_{50}$, d.h. die Konzentration, welche eine Hemmung von 50 % der Kontrollaktivität bewirkt. Die $IC_{50}$ wird graphisch ermittelt durch Auftragen der prozentualen Hemmung auf der Ordinate gegen den log der Konzentration (µM) auf der Abszisse.

Unter den beschriebenen Versuchsbedingungen hemmt Mepacrin die Phospholipase $A_2$ mit einer $IC_{50}$ von 1600 µM.

In vitro-Test zur Bestimmung der Hemmung der Phospholipase C aus menschlichen Thrombozyten

Menschliche Thrombozyten werden aus "Buffy coats" durch fraktionierte Zentrifugation gewonnen und anschliessend tiefgefroren. Die Phospholipase C wird durch Ultraschallbehandlung der Zellsuspension freigesetzt und findet sich nach Ultrazentrifugation (150'000 x g während 1 Stunde) in löslicher Form im Ueberstand.

Für die Bestimmung der Enzymaktivität wird Enzym (20-100 µg Protein) in 0.025 M Tris/Maleat-Puffer pH 6 unter Zusatz von 0.2 mM $CaCl_2$ und 0.02 mM radioaktiv markiertem Substrat, Phosphatidyl-[$^{14}C$]-inosit, bei 37° während 5 Minuten inkubiert. Die Reaktion wird abgestoppt durch Ausschütteln mit $CHCl_3/CH_3OH$ 2:1 (v/v). Dabei wird unverbrauchtes Substrat in die organische Phase extrahiert, während das Reaktionsprodukt $^{14}C$-Inositphophat in der wässrigen Phase verbleibt und durch Radiometrie eines Aliquots gemessen werden kann.

Zur Ermittlung einer Hemmwirkung von Prüfsubstanzen auf die Phospholipase C werden diese als Lösungen in Wasser, Dimethylsulfoxid (Endkonzentration im Ansatz bis 5 %, v/v) oder Ethanol (Endkonzentration im Ansatz bis 2.5 %, v/v) dem Inkubationsansatz zugesetzt. Die Wirkungsstärke der Prüfsubstanzen wird ausgedrückt durch die $IC_{50}$, d.h. die Konzentration, welche eine Hemmung von 50 % der Kontrollaktivität bewirkt. Die $IC_{50}$ wird graphisch ermittelt durch Auftragen der prozentualen Hemmung auf der Ordinate gegen den log der Konzentration (µM) auf der Abszisse.

Unter den beschriebenen Versuchsbedingungen hemmt Mepacrin die Phospholipase C mit einer $IC_{50}$

von 20 µM.

**Patentansprüche**

**Patentansprüche für folgende Vertragsstaaten: DE, GB, FR, CH, LI, IT, NL, BE, SE, LU**

1. Eine Verbindung der Formel

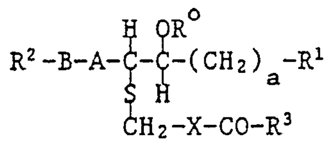

(I)

worin $R^0$ Wasserstoff oder $C_1$-$C_7$-Alkanoyl bedeutet, $R^1$ $C_1$-$C_3$-Alkyl, welches am endständigen C-Atom durch Hydroxy, $C_1$-$C_7$-Alkanoyloxy, Benzoyloxy, Halogen der Atomnummer bis und mit 17 oder Methoxy substituiert sein kann, oder $C_1$-$C_3$-Perfluoralkyl darstellt, $R^2$ einen aliphatischen Rest mit 5 bis 15 C-Atomen bedeutet, $R^3$ Hydroxy, $C_1$-$C_7$-Alkoxy oder eine Gruppe der Formel

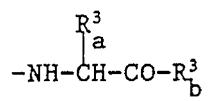

(Ia)

darstellt, in der $R^3_a$ Wasserstoff oder $C_1$-$C_5$-Alkyl und $R^3_b$ Hydroxy, $C_1$-$C_7$-Alkoxy oder Amino bedeutet, A Ethylen bedeutet oder, falls $R^1$ einen halogenierten Rest und/oder B Phenylen darstellt, Vinylen oder eine Einfachbindung darstellt, B Phenylen oder eine Einfachbindung bedeutet, a für eine ganze Zahl von 1 bis 7 steht und X eine Einfachbindung, die Methylengruppe oder eine unsubstituierte oder durch den Acylrest einer gegebenenfalls mono- oder polyhalogenierten $C_1$-$C_5$-Alkansäure oder durch $\gamma$-Glutamyl N-substituierte Aminomethylengruppe bedeutet, mit der Massgabe, dass $R_1$ von Hydroxymethyl verschieden ist, wenn B eine Einfachbindung bedeutet, sowie Salze solcher Verbindungen mit salzbildenden Eigenschaften.

2. Eine Verbindung gemäss Anspruch 1, worin in Formel I a = 3-6 ist, $R^0$ Wasserstoff, $R^1$ Trifluormethyl, $R^2$-B-A zusammen ein lineares $C_{10-16}$-1-Alkenyl, $R^3$ Hydroxyl, $C_{1-7}$-Alkoxy oder den Rest der Partialformel -NH-$CH_2$-COR (wobei R Hydroxyl oder $C_{1-7}$-Alkoxy ist), und -X- die Methylengruppe oder die Gruppe der Partialformel

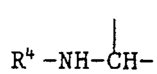

darstellt, worin $R^4$ Wasserstoff, Acetyl oder Trifluoracetyl bedeutet, sowie Salze von Verbindungen mit salzbildenden Eigenschaften.

3. Eine Verbindung gemäss Anspruch 2, worin in Formel I a, $R^0$, $R^1$, A, B und $R^2$ die dort angegebenen Bedeutungen haben und die Gruppierung -S-$CH_2$-X-CO-$R^3$ einen gegebenenfalls N-acylierten N-(L-Cysteinyl)-glycyl-Rest der Formel

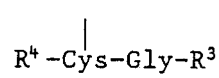

,

in welcher $R^3$ Hydroxyl oder $C_{1-4}$-Alkoxy und $R^4$ Wasserstoff, Acetyl oder Trifluoracetyl ist, darstellt, sowie Alkalimetallsalze von Verbindungen mit salzbildenden Eigenschaften.

**4.** Eine Verbindung gemäss Anspruch 1, worin in Formel I a = 3-6, $R^0$ Wasserstoff, $R^1$ Methyl, Chlormethyl oder Fluormethyl, A eine Einfachbindung, B Phenylen, $R^2$ ein lineares $C_{5-15}$-Alkyl, $R^3$ Hydroxyl oder den Rest der Partialformel $-NH-CH_2-COOH$ und -X- die Methylengruppe oder die Gruppe der Partialformel

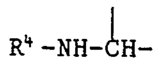

darstellt, worin $R^4$ Wasserstoff, Acetyl oder Trifluoracetyl bedeutet, sowie ihre Salze.

**5.** Eine Verbindung gemäss Anspruch 4, worin in Formel I a, $R^0$, $R^1$, A, B und $R^2$ die dort angegebenen Bedeutungen haben und die Gruppierung $-S-CH_2-X-CO-R^3$ einen durch das S-Atom gebundenen Rest der 3-Mercaptopropionsäure oder ihres Alkalimetallsalzes darstellt.

**6.** Ein physiologisch verträgliches Alkalimetallsalz einer solchen Verbindung gemäss einem der Ansprüche 1-5, welche mindestens eine freie Carboxylgruppe aufweist.

**7.** Eine Verbindung gemäss einem der Ansprüche 1-6 zur Verwendung als Leukotrien-Antagonist und/oder zur Hemmung von Phospholipasen.

**8.** Verfahren zur Herstellung von Verbindungen der in Anspruch 1 definierten Formel I, dadurch gekennzeichnet, dass man ein Epoxid der Formel

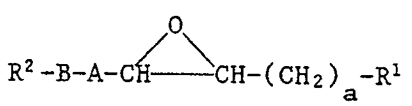

(II)

worin a, A, B, $R^1$ und $R^2$ die im Anspruch 1 angegebenen Bedeutungen haben und in welchem eine gegebenenfalls vorhandene Hydroxylgruppe in einer geschützten Form vorliegen kann, mit einem Mercaptoalkancarbonsäure-Derivat der Formel

$HS-CH_2-X-CO-R^3$ (III)

worin $R^3$ und -X- die im Anspruch 1 angegebenen Bedeutungen haben und in welchem eine gegebenenfalls vorhandene Aminogruppe in einer geschützten Form vorliegen kann, umsetzt und wenn notwendig oder erwünscht, eine erhaltene Verbindung der Formel I, worin $R^0$ Wasserstoff bedeutet, zu einer entsprechenden Verbindung, worin $R^0$ ein $C_{1-7}$-Alkanoyl ist, acyliert, und/oder die Schutzgruppe(n) der Hydroxyl- und/oder Aminogruppe(n) abspaltet und/oder eine als Ester vorliegende Verbindung zur freien Säure oder einem Salz verseift und/oder eine erhaltene freie Verbindung mit salzbildenden Eigenschaften in ein Salz umwandelt und/oder eine Verbindung aus einer entsprechenden Salzform freisetzt.

**9.** Pharmazeutische Zusammensetzungen enthaltend als Wirkstoff mindestens eine der Verbindungen gemäss einem der Ansprüche 1-7 zusammen mit mindestens einem pharmazeutisch annehmbaren Trägermaterial.

**10.** Verwendung der Verbindungen gemäss einem der Ansprüche 1-7 zur Herstellung einer pharmazeutischen Zusammensetzung, welche zur Linderung oder Behebung der krankhaften Zustände oder Symptome in einem Säuger, welche auf die allergogene Einwirkung von Leukotrienen oder eine Entzündung zurückzuführen sind, anwendbar ist.

**Patentansprüche für folgende Vertragsstaaten: AT, ES, GR**

1. Verfahren zur Herstellung einer Verbindung der Formel

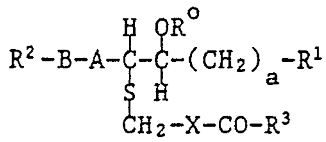

(I)

worin $R^0$ Wasserstoff oder $C_1$-$C_7$-Alkanoyl bedeutet, $R^1$ $C_1$-$C_3$-Alkyl, welches am endständigen C-Atom durch Hydroxy, $C_1$-$C_7$-Alkanoyloxy, Benzoyloxy, Halogen der Atomnummer bis und mit 17 oder Methoxy substituiert sein kann, oder $C_1$-$C_3$-Perfluoralkyl darstellt, $R^2$ einen aliphatischen Rest mit 5 bis 15 C-Atomen bedeutet, $R^3$ Hydroxy, $C_1$-$C_7$-Alkoxy oder eine Gruppe der Formel

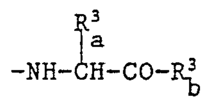

(Ia)

darstellt, in der $R^3$ Wasserstoff oder $C_1$-$C_5$-Alkyl und $R^3_b$ Hydroxy, $C_1$-$C_7$-Alkoxy oder Amino bedeutet, A Ethylen bedeutet oder, falls $R^1$ einen halogenierten Rest und/oder B Phenylen darstellt, Vinylen oder eine Einfachbindung darstellt, B Phenylen oder eine Einfachbindung bedeutet, a für eine ganze Zahl von 1 bis 7 steht und X eine Einfachbindung, die Methylengruppe oder eine unsubstituierte oder durch den Acylrest einer gegebenenfalls mono- oder polyhalogenierten $C_1$-$C_5$-Alkansäure oder durch $\gamma$-Glutamyl N-substituierte Aminomethylengruppe bedeutet, mit der Massgabe, dass $R_1$ von Hydroxymethyl verschieden ist, wenn B eine Einfachbindung bedeutet,
sowie von Salzen solcher Verbindungen mit salzbildenden Eigenschaften, dadurch gekennzeichnet, dass man ein Epoxid der Formel

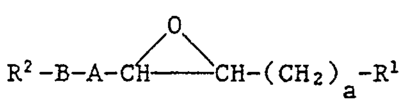

(II)

worin a, A, B, $R^1$ und $R^2$ die oben angegebenen Bedeutungen haben und in welchem eine gegebenenfalls vorhandene Hydroxylgruppe in einer geschützten Form vorliegen kann, mit einem Mercaptoalkancarbonsäure-Derivat der Formel

$HS-CH_2-X-CO-R^3$ (III)

worin $R^3$ und -X- die oben angegebenen Bedeutungen haben und in welchem eine gegebenenfalls vorhandene Aminogruppe in einer geschützten Form vorliegen kann, umsetzt und wenn notwendig oder erwünscht, eine erhaltene Verbindung der Formel I, worin $R^0$ Wasserstoff bedeutet, zu einer entsprechenden Verbindung, worin $R^0$ ein $C_{1-7}$-Alkanoyl ist, acyliert, und/oder die Schutzgruppe(n) der Hydroxyl- und/oder Aminogruppe(n) abspaltet und/oder eine als Ester vorliegende Verbindung zur freien Säure oder einem Salz verseift und/oder eine erhaltene freie Verbindung mit salzbildenden Eigenschaften in ein Salz umwandelt und/oder eine Verbindung aus einer entsprechenden Salzform freisetzt.

2. Ein Verfahren gemäss Anspruch 1, dadurch gekennzeichnet, dass man eine Verbindung der Formel I, worin a = 3-6 ist, $R^0$ Wasserstoff, $R^1$ Trifluormethyl, $R^2$-B-A zusammen ein lineares $C_{10-16}$-1-Alkenyl, $R^3$ Hydroxyl, $C_{1-7}$-Alkoxy oder den Rest der Partialformel $-NH-CH_2-COR$ (wobei R Hydroxyl oder $C_{1-7}$-Alkoxy ist), und -X- die Methylengruppe oder die Gruppe der Partialformel

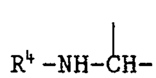

darstellt, worin $R^4$ Wasserstoff, Acetyl oder Trifluoracetyl bedeutet, sowie Salze von Verbindungen mit salzbildenden Eigenschaften herstellt.

3. Ein Verfahren gemäss Anspruch 2, dadurch gekennzeichnet, dass man eine Verbindung der Formel I, worin a, $R^0$, $R^1$, A, B und $R^2$ die dort angegebenen Bedeutungen haben und die Gruppierung -S-$CH_2$-X-CO-$R^3$einen gegebenenfalls N-acylierten N-(L-Cysteinyl)-glycyl-Rest der Formel

$R^4$-Cys-Gly-$R^3$ ,

in welcher $R^3$ Hydroxyl oder $C_{1-4}$-Alkoxy und $R^4$ Wasserstoff, Acetyl oder Trifluoracetyl ist, darstellt, sowie Alkalimetallsalze von Verbindungen mit salzbildenden Eigenschaften herstellt.

4. Ein Verfahren gemäss Anspruch 1, dadurch gekennzeichnet, dass man eine Verbindung der Formel I, worin a = 3-6, $R^0$ Wasserstoff, $R^1$ Methyl, Chlormethyl oder Fluormethyl, A eine Einfachbindung, B Phenylen, $R^2$ ein lineares $C_{5-15}$-Alkyl, $R^3$ Hydroxyl oder den Rest der Partialformel -NH-$CH_2$-COOH und -X- die Methylengruppe oder die Gruppe der Partialformel

$R^4$-NH-CH-

darstellt, worin $R^4$ Wasserstoff, Acetyl oder Trifluoracetyl bedeutet, sowie ihre Salze herstellt.

5. Ein Verfahren gemäss Anspruch 4, dadurch gekennzeichnet, dass man eine Verbindung der Formel I, worin a, $R^0$, $R^1$, A, B und $R^2$ die dort angegebenen Bedeutungen haben und die Gruppierung -S-$CH_2$-X-CO-$R^3$einen durch das S-Atom gebundenen Rest der 3-Mercaptopropionsäure oder ihres Alkalimetallsalzes darstellt, herstellt.

6. Verfahren gemäss einem der Ansprüche 1-5, dadurch gekennzeichnet, dass man ein physiologisch verträgliches Alkalimetallsalz einer der dort charakterisierten Verbindungen mit mindestens einer freien Carboxylgruppe herstellt.

7. Verfahren gemäss einem der Ansprüche 1-6, dadurch gekennzeichnet, dass man eine Verbindung der dort definierten Formel I zur Verwendung als Leukotrien-Antagonist und/oder zur Hemmung von Phospholipasen herstellt.

8. Verfahren zur Herstellung einer pharmazeutischen Zusammensetzung enthaltend als Wirkstoff mindestens eine der gemäss einem der Ansprüche 1-7 hergestellten Verbindungen der Formel I zusammen mit mindestens einem pharmazeutisch annehmbaren Trägermaterial, dadurch gekennzeichnet, dass man die entsprechenden Bestandteile auf nicht-chemischem Wege vermischt und, wenn eine fertige Arzneimittelform erwünscht ist, in geeignete Behälter in abgemessenen Mengen abfüllt.

9. Verwendung der gemäss einem der Ansprüche 1-7 hergestellten Verbindungen zur Herstellung einer pharmazeutischen Zusammensetzung, welche zur Linderung oder Behebung der krankhaften Zustände oder Symptome in einem Säuger, welche auf die allergogene Einwirkung von Leukotrienen oder eine Entzündung zurückzuführen sind, anwendbar ist.

**Claims**

**Claims for the following Contracting states: DE, GB, FR, CH, LI, IT, NL, BE, SE, LU**

1. A compound of the formula

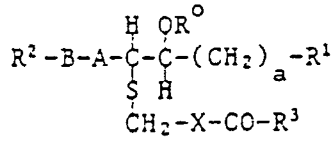

(I)

wherein $R^o$ is hydrogen or $C_1$-$C_7$-alkanoyl, $R^1$ is $C_1$-$C_3$alkyl that may be substituted at the terminal carbon atom by hydroxy, $C_1$-$C_7$alkanoyloxy, benzoyloxy, halogen having an atomic number of up to and including 17 or by methoxy, or is $C_1$-$C_3$perfluoroalkyl, $R^2$ is an aliphatic radical having from 5 to 15 carbon atoms, $R^3$ is hydroxy, $C_1$-$C_7$alkoxy or a group of the formula

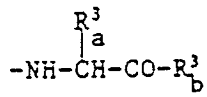

(Ia)

in which $R^3_a$ is hydrogen or $C_1$-$C_5$alkyl and $R^3_b$ is hydroxy, $C_1$-$C_7$alkoxy or amino, A is ethylene or, if $R^1$ is a halogenated radical and/or B is phenylene, is vinylene or a single bond, B is phenylene or a single bond, a is an integer from 1 to 7 and X is a single bond, a methylene group, or an aminomethylene group that is unsubstituted or N-substituted by the acyl radical of an unsubstituted or mono- or Polyhalogenated $C_1$-$C_5$alkanoic acid or by $\gamma$-glutamyl, with the proviso that $R_1$ is other than hydroxymethyl when B is a single bond, or a salt of such a compound having salt-forming properties.

2. A compound according to claim 1, wherein in formula I a = 3-6, $R^o$ is hydrogen, $R^1$ is trifluoromethyl, $R^2$-B-A together are a linear $C_{10-16}$-1-alkenyl, $R^3$ is hydroxy, $C_{1-7}$alkoxy or a radical of the partial formula -NH-$CH_2$-COR (in which R is hydroxy or $C_{1-7}$alkoxy), and -X- is a methylene group or a group of the partial formula

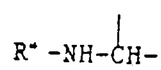

in which $R^4$ is hydrogen, acetyl or trifluoroacetyl, or a salt of such a compound having salt-forming properties.

3. A compound according to claim 2, wherein in formula I a, $R^o$, $R^1$, A, B and $R^2$ have the meanings given therein and the grouping -S-$CH_2$-X-CO-$R^3$ is an unsubstituted or N-acylated N-(L-cysteinyl)-glycyl radical of the formula

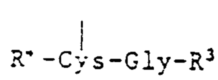

,

in which $R^3$ is hydroxy or $C_{1-4}$alkoxy and $R^4$ is hydrogen, acetyl or trifluoroacetyl, or an alkali metal salt of such a compound having salt-forming properties.

4. A compound according to claim 1, wherein in formula I a = 3-6, $R^o$ is hydrogen, $R^1$ is methyl, chloromethyl or fluoromethyl, A is a single bond, B is phenylene, $R^2$ is a linear $C_{5-15}$alkyl, $R^3$ is hydroxy or a radical of the partial formula -NH-$CH_2$-COOH and -X- is a methylene group or a group of the partial formula

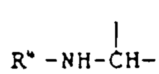

in which $R^4$ is hydrogen, acetyl or trifluoroacetyl, or a salt thereof.

5. A compound according to claim 4, wherein in formula I a, $R^o$, $R^1$, A, B and $R^2$ have the meanings given therein and the grouping $-S-CH_2-X-CO-R^3$ is a radical of 3-mercaptopropionic acid that is bonded by the S atom, or an alkali metal salt thereof.

6. A physiologically tolerable alkali metal salt of a compound according to any one of claims 1 to 5 that contains at least one free carboxy group.

7. A compound according to any one of claims 1 to 6 for use as a leucotriene antagonist and/or for the inhibition of phospholipases.

8. A process for the manufacture of a compound of the formula I defined in claim 1, which comprises reacting an epoxide of the formula

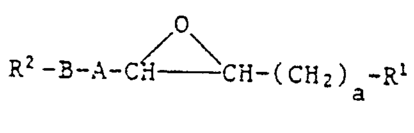

(II)

in which a, A, B, $R^1$ and $R^2$ have the meanings given in claim 1 and in which a hydroxy group, if present, can be in a protected form, with a mercaptoalkanecarboxylic acid derivative of the formula

$HS-CH_2-X-CO-R^3$ (III)

in which $R^3$ and -X- have the meanings given in claim 1 and in which an amino group, if present, can be in a protected form, and, if necessary or desired, acylating a resulting compound of the formula I in which $R^o$ is hydrogen to a corresponding compound in which $R^o$ is $C_{1-7}$alkanoyl, and/or removing the protecting group(s) of the hydroxy and/or amino group(s), and/or hydrolysing a compound present in the form of an ester to the free acid or a salt, and/or converting a resulting free compound having salt-forming properties into a salt, and/or freeing a compound from a corresponding salt form.

9. A pharmaceutical composition comprising as active ingredient at least one of the compounds according to any one of claims 1 to 7 together with at least one pharmaceutically acceptable carrier.

10. The use of compounds according to any one of claims 1-7 for the manufacture of a pharmaceutical composition that can be used for the alleviation or elimination of pathological conditions or symptoms in a mammal that are attributable to the allergogenic action of leucotrienes or an inflammation.

**Claims for the following Contracting States: AT, ES, GR.**

1. A process for the manufacture of a compound of the formula

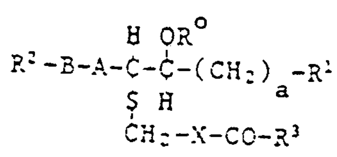

(I)

wherein $R^o$ is hydrogen or $C_1$-$C_7$alkanoyl, $R^1$ is $C_1$-$C_3$alkyl that may be substituted at the terminal carbon atom by hydroxy, $C_1$-$C_7$alkanoyloxy, benzoyloxy, halogen having an atomic number of up to and including 17 or by methoxy, or is $C_1$-$C_3$perfluoroalkyl, $R^2$ is an aliphatic radical having from 5 to 15 carbon atoms, $R^3$ is hydroxy, $C_1$-$C_7$alkoxy or a group of the formula

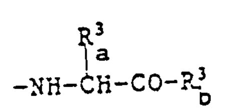

(Ia)

in which $R^3_a$ is hydrogen or $C_1$-$C_5$alkyl and $R^3_b$ is hydroxy, $C_1$-$C_7$alkoxy or amino, A is ethylene or, if $R^1$ is a halogenated radical and/or B is phenylene, is vinylene or a single bond, B is phenylene or a single bond, a is an integer from 1 to 7 and X is a single bond, a methylene group, or an aminomethylene group that is unsubstituted or N-substituted by the acyl radical of an unsubstituted or mono- or poly-halogenated $C_1$-$C_5$alkanoic acid or by $\gamma$-glutamyl, with the proviso that $R_1$ is other than hydroxymethyl when B is a single bond, or a salt of such a compound having salt-forming properties, which comprises reacting an epoxide of the formula

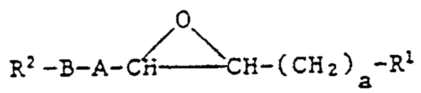

(II)

in which a, A, B, $R^1$ and $R^2$ have the meanings given above and in which a hydroxy group, if present, can be in a protected form, with a mercaptoalkanecarboxylic acid derivative of the formula

$HS$-$CH_2$-X-CO-$R^3$ (III)

in which $R^3$ and -X- have the meanings given above and in which an amino group, if present, can be in a protected form, and, if necessary or desired, acylating a resulting compound of the formula I in which $R^o$ is hydrogen to a corresponding compound in which $R^o$ is $C_{1-7}$alkanoyl, and/or removing the protecting group(s) of the hydroxy and/or amino group(s), and/or hydrolysing a compound present in the form of an ester to the free acid or a salt, and/or converting a resulting free compound having salt-forming properties into a salt, and/or freeing a compound from a corresponding salt form.

**2.** A process according to claim 1, wherein a compound of the formula I in which a = 3-6, $R^o$ is hydrogen, $R^1$ is trifluoromethyl, $R^2$-B-A together are a linear $C_{10-16}$-1-alkenyl, $R^3$ is hydroxy, $C_{1-7}$alkoxy or a radical of the partial formula -NH-$CH_2$-COR (in which R is hydroxy or $C_{1-7}$alkoxy), and -X- is a methylene group or a group of the partial formula

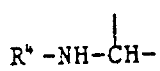

in which $R^4$ is hydrogen, acetyl or trifluoroacetyl, or a salt of such a compound having salt-forming properties, is manufactured.

**3.** A process according to claim 2, wherein a compound of the formula I in which a, $R^o$, $R^1$, A, B and $R^2$ have the meanings given therein and the grouping -S-$CH_2$-X-CO-$R^3$ is an unsubstituted or N-acylated N-(L-cysteinyl)-glycyl radical of the formula

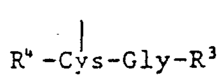

,

in which $R^3$ is hydroxy or $C_{1-4}$alkoxy and $R^4$ is hydrogen, acetyl or trifluoroacetyl, or an alkali metal salt of such a compound having salt-forming properties, is manufactured.

**4.** A process according to claim 1, wherein a compound of the formula I in which a = 3-6, $R^o$ is hydrogen, $R^1$ is methyl, chloromethyl or fluoromethyl, A is a single bond, B is phenylene, $R^2$ is a linear $C_{5-15}$alkyl, $R^3$ is hydroxy or a radical of the partial formula -NH-$CH_2$-COOH and -X-is a methylene group or a group of the partial formula

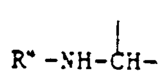

in which $R^4$ is hydrogen, acetyl or trifluoroacetyl, or a salt thereof, is manufactured.

**5.** A process according to claim 4, wherein a compound of the formula I in which a, $R^{o}$, $R^1$, A, B and $R^2$ have the meanings given therein and the grouping $-S-CH_2-X-CO-R^3$ is a radical of 3-mercaptopropionic acid bonded by the S atom, or an alkali metal salt thereof, is manufactured.

**6.** A process according to any one of claims 1 to 5, wherein a physiologically tolerable alkali metal salt of one of the compounds characterised therein that contains at least one free carboxy group is manufactured.

**7.** A process according to any one of claims 1 to 6, wherein a compound of the formula I defined therein is manufactured for use as a leucotriene antagonist and/or for the inhibition of phospholipases.

**8.** A process for the manufacture of a pharmaceutical composition comprising as active ingredient at least one of the compounds of the formula I manufactured according to any one of claims 1 to 7 together with at least one pharmaceutically acceptable carrier, wherein the appropriate components are mixed by non-chemical methods and, if a finished medicament form is desired, filled into suitable containers in measured amounts.

**9.** The use of a compound manufactured in accordance with any one of claims 1-7 for the manufacture of a pharmaceutical composition that can be used for the alleviation or elimination of pathological conditions or symptoms in a mammal that are attributable to the allergogenic action of leucotrienes or an inflammation.

## Revendications

**Revendications pour les Etats contractants suivants: DE, GB, FR, CH, LI, IT, NL, BE, SE, LU**

**1.** Composé de formule

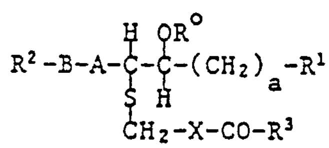

(I)

où $R^0$ représente l'hydrogène ou un alcanoyle en $C_1$-$C_7$, $R^1$ désigne un alkyle en $C_1$-$C_3$, qui peut être substitué sur l'atome de carbone terminal par un radical hydroxy, alcanoyloxy en $C_1$-$C_7$, benzoyloxy, halogène ayant un numéro atomique allant jusqu'à 17 inclus ou méthoxy, ou un perfluoroalkyle en $C_1$-$C_3$, $R^2$ représente un reste aliphatique ayant de 5 à 15 atomes de carbone, $R^3$ est un hydroxy, un alcoxy en $C_1$-$C_7$ ou un groupe de formule

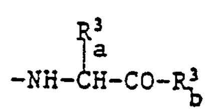

(Ia)

dans laquelle $R^3_a$ est l'hydrogène ou un alkyle en $C_1$-$C_5$ et $R^3_b$ désigne un hydroxy, un alcoxy en $C_1$-$C_7$ ou un amino, A désigne l'éthylène ou, dans le cas ou $R^1$ est un reste halogéné et/ou B est un phénylène, A désigne un vinylène ou une liaison simple, B désigne un phénylène ou une liaison simple, a désigne un nombre entier de 1 à 7 et X représente une liaison simple, le groupe méthylène ou un groupe aminométhylène non substitué ou N-substitué par le reste acyle d'un acide alcanoïque en $C_1$-$C_5$ éventuellement mono- ou polyhalogéné ou par un $\gamma$-glutamyle, à la condition que $R^1$ ne soit pas l'hydroxyméthyle quand B représente une liaison simple, ainsi que les sels de ces composés qui peuvent être salifiés.

**2.** Composé selon la revendication 1, dans lequel dans la formule I a = 3-6, $R^0$ est l'hydrogène, $R^1$ représente le trifluorométhyle, $R^2$-B-A désignent ensemble un 1-alcényle linéaire en $C_{10-16}$, $R^3$ désigne

l'hydroxyle, un alcoxy en $C_{1-7}$ ou le reste de formule partielle -NH-$CH_2$-COR (où R est l'hydroxyle ou un alcoxy en $C_{1-7}$), et -X-est le groupe méthylène ou le groupe de formule partielle

$$R^4-NH-\overset{|}{C}H-$$

où $R^4$ désigne l'hydrogène, un acétyle ou un trifluoroacétyle, ainsi que les sels de ces composés qui peuvent être salifiés.

**3.** Composé selon la revendication 2, dans lequel,dans la formule I,a $R^0$, $R^1$, A, B et $R^2$ ont les significations alors indiquées et le groupement -S-$CH_2$-X-CO-$R^3$ désigne un reste N-(L-cystéinyl)-glycyle éventuellement N-acylé de formule

$$R^4-\overset{|}{Cys}-Gly-R^3 \quad ,$$

où $R^3$ est l'hydroxyle ou un alcoxy en $C_{1-4}$ et $R^4$ est l' hydrogène, un acétyle ou un trifluoroacétyle, ainsi que les sels de métal alcalin de ces composés qui peuvent être salifiés.

**4.** Composé selon la revendication 1, dans lequel dans la formule I a = 3-6, $R^0$ est l'hydrogène, $R^1$ désigne un méthyle, un chlorométhyle ou un fluorométhyle, A est une liaison simple, B représente un phénylène, $R^2$ désigne un alkyle linéaire en $C_{5-15}$, $R^3$ désigne un hydroxyle ou le reste de formule partielle -NH-$CH_2$-COOH et -X- désigne le groupe méthylène ou le groupe de formule partielle

$$R^4-NH-\overset{|}{C}H-$$

où $R^4$ est l'hydrogène, un acétyle ou un trifluoroacétyle, ainsi que leurs sels.

**5.** Composé selon la revendication 4, dans lequel dans la formule I a, $R^0$, $R^1$, A, B et $R^2$ ont les significations alors indiquées et le groupement -S-$CH_2$-X-CO-$R^3$ désigne un reste lié par l'atome S de l'acide 3-mercaptopropionique ou son sel de métal alcalin.

**6.** Sel de métal alcalin physiologiquement acceptable d' composé selon l'une quelconque des revendications 1-5 qui présente au moins un groupe carboxyle libre.

**7.** Composé selon l'une quelconque des revendications 1-6 pour l'utilisation comme antagoniste du leucotriène et/ou pour l'inhibition des phospholipases.

**8.** Procédé pour la préparation de composés de formule I définie dans la revendication 1, caractérisé en ce qu'on fait réagir un époxyde de formule

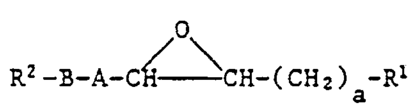

(II)

où a, A, B, $R^1$ et $R^2$ ont les significations indiquées dans la revendication 1 et où un groupe hydroxyle éventuellement présent peut figurer sous une forme protégée, avec un dérivé d'acide mercaptoalcane-carboxylique de formule

HS-$CH_2$-X-CO-$R^3$ (III)

où $R^3$ et -X- ont les significations indiquées dans la revendication 1 et où un groupe amino éventuellement présent peut être présent sous une forme protégée, et si nécessaire ou souhaité on acyle un composé obtenu de formule I, où $R^0$est l'hydrogène, en un composé correspondant dans lequel $R^0$ est un alcanoyle en $C_{1-7}$, et/ou on élimine le(s) groupe(s) protecteur(s) du(des) groupe(s) hydroxyle et/ou amino, et/ou on saponifie un composé présent sous forme d'ester pour former l'acide libre ou un sel de celui-ci, et/ou on transforme un composé libre obtenu ayant des propriétés salifiantes en un sel et/ou on libère un composé à partir de la forme sel correspondante.

**9.** Compositions pharmaceutiques contenant comme principe actif au moins un des composés selon l'une quelconque des revendications 1-7, conjointement avec au moins une matière support pharmaceutiquement acceptable.

**10.** Utilisation de composés selon l'une quelconque des revendications 1-7 pour la préparation d'une composition pharmaceutique utilisable pour le soulagement ou la suppression d'états maladifs ou de symptômes maladifs d' un mammifère, qui peuvent être attribués à l'action allergogène des leucotriènes ou à une inflammation.

**Revendications pour les Etats contractants suivants: AT, ES, GR**

**1.** Procédé pour la préparation d'un composé de formule

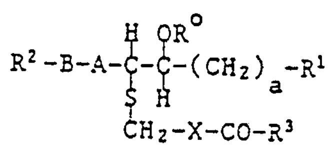

(I)

où $R^0$ représente l'hydrogène ou un alcanoyle en $C_1$-$C_7$, $R^1$ désigne un alkyle en $C_1$-$C_3$, qui peut être substitué sur l'atome de carbone terminal par un radical hydroxy, alcanoyloxy en $C_1$-$C_7$, benzoyloxy, halogène ayant un numéro atomique allant jusqu'à 17 inclus ou méthoxy, ou un perfluoroalkyle en $C_1$-$C_3$, $R^2$ représente un reste aliphatique ayant de 5 à 15 atomes de carbone, $R^3$ est un hydroxy, un alcoxy en $C_1$-$C_7$ ou un groupe de formule

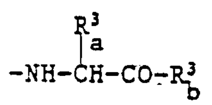

(Ia)

dans laquelle $R^3_a$ est l'hydrogène ou un alkyle en $C_1$-$C_5$ et $R^3_b$ désigne un hydroxy, un alcoxy en $C_1$-$C_7$ ou un amino, A désigne l'éthylène ou, dans le cas où $R^1$ est un reste halogéné et/ou B est un phénylène, A désigne un vinylène ou une liaison simple, B désigne un phénylène ou une liaison simple, a désigne un nombre entier de 1 à 7 et X représente une liaison simple, le groupe méthylène ou un groupe aminométhylène non substitué ou N-substitué par le reste acyle d'un acide alcanoïque en $C_1$-$C_5$ éventuellement mono- ou pokyhalogéné ou par un $\gamma$-glutamyle, à la condition que $R^1$ ne soit pas l'hydroxyméthyle quand B représente une liaison simple, ainsi que les sels de ces composés qui peuvent être salifiés, caractérisé en ce qu'on fait réagir un époxyde de formule

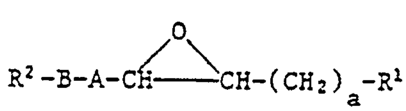

(II)

où a, A, B, $R^1$ et $R^2$ ont les significations indiquées plus haut et où un groupe hydroxyle éventuellement présent peut figurer sous une forme protégée, avec un dérivé d'acide mercaptoalcanecarboxylique de

formule

$HS-CH_2-X-CO-R^3$ (III)

où $R^3$ et -X- ont les significations indiquées plus haut et où un groupe amino éventuellement présent peut être présent sous une forme protégée, et si nécessaire ou souhaité on acyle un composé obtenu de formule I, où $R^0$ est l'hydrogène, en un composé correspondant dans lequel $R^0$ est un alcanoyle en $C_{1-7}$, et/ou on élimine le(s) groupe(s) protecteur(s) du(des) groupe(s) hydroxyle et/ou amino, et/ou on saponifie un composé présent sous forme d'ester pour former l'acide libre ou un sel de celui-ci, et/ou on transforme un composé libre obtenu ayant des propriétés salifiantes en un sel et/ou on libère un composé à partir de la forme sel correspondante

2. Procédé selon la revendication 1, caractérisé en ce qu'on prépare un composé de formule I où a = 3-6, $R^0$ est l'hydrogène, $R^1$ représente le trifluorométhyle, $R^2$-B-A désignent ensemble un I-alcényle linéaire en $C_{10-16}$, $R^3$ désigne l'hydroxyle, un alcoxy en $C_{1-7}$ ou le reste de formule partielle $-NH-CH_2-COR$ (où R est l'hydroxyle ou un alcoxy en $C_{1-7}$), et -X- est le groupe méthylène ou le groupe de formule partielle

$R^4$ -NH-CH-

où $R^4$ désigne l'hydrogène un acétyle ou un trifluoroacétyle, ainsi que les sels de ces composés qui peuvent être salifiés.

3. Procédé selon la revendication 2, caractérisé en ce qu'on prépare un composé de formule I où a, $R^0$, $R^1$, A, B et $R^2$ ont les significations qui y sont indiquées et le groupement $-S-CH_2-X-CO-R^3$ désigne un reste N-(L-cystéinyl)-glycyle éventuellement N-acylé de formule

$R^4$ -Cys-Gly-$R^3$ ,

où $R^3$ est l'hydroxyle ou un alcoxy en $C_{1-4}$ et $R^4$ est l' hydrogène, un acétyle ou un trifluoroacétyle, ainsi que les sels de métal alcalin de ces composés qui peuvent être salifiés.

4. Procédé selon la revendication 1, caractérisé en ce qu'on prépare un composé de formule I ou a = 3-6, $R^0$ est l'hydrogène, $R^1$ désigne un méthyle, un chlorométhyle ou un fluorométhyle, A est une liaison simple, B représente un phénylène, $R^2$ désigne un alkyle linéaire en $C_{5-15}$, $R^3$ désigne un hydroxyle ou le reste de formule partielle $-NH-CH_2-COOH$ et -X- désigne le groupe méthylène ou le groupe de formule partielle

$R^4$ -NH-CH-

ou $R^4$ est l'hydrogène, un acétyle ou un trifluoroacétyle, ainsi que leurs sels.

5. Procédé selon la revendication 4, caractérisé en ce qu'on prépare un composé de formule I où a, $R^0$, $R^1$, A, B et $R^2$ ont les significations qui y sont indiquées et le groupement $-S-CH_2-X-CO-R^3$ désigne un reste lié par l' atome S de l'acide 3-mercaptopropionique ou son sel de métal alcalin.

6. Procédé selon l'une quelconque des revendications 1-5, caractérisé en ce qu'on prépare un sel de métal alcalin physiologiquement acceptable d'un des composés qui y sont caractérisés ayant au moins un groupe carboxyle libre.

7. Procédé selon l'une quelconque des revendications 1-6, caractérisé en ce qu'on prépare un composé répondant à la formule I qui y est définie pour l'utilisation comme antagoniste du leucotriène et/ou pour l'inhibition des phospholipases.

8. Procédé pour la préparation d'une composition pharmaceutique contenant comme principe actif au moins un des composés de formule I préparés selon l'une quelconque des revendications 1-7 conjointement avec au moins une matière support pharmaceutiquement acceptable. caractérisé en ce qu'on mélange les composants correspondants par une voie non chimique et, lorsqu'on souhaite un médicament sous forme finie, on verse dans des récipients appropriés en des quantités mesurées.

9. Utilisation des composés préparés selon l'une quelconque des revendications 1-7 pour la préparation d'une composition pharmaceutique utilisable pour le soulagement ou la suppression des états maladifs ou des symptômes maladifs d'un mammifère, qui peuvent être attribués à l'action allergogène des leucotriènes ou à une inflammation.